# EUROPEAN PATENT APPLICATION

(11) **EP 2 502 924 A1**
(43) Date of publication of application: **26.09.2012**
(21) Application number: 11159589.8
(22) Date of filing: 24.03.2011
(51) Int. Cl.: C07D 403/14, A61K 31/506, A61P 35/00

(54) **Novel pyrimidine derivatives**

(71) Applicant: Chemilia AB, 141 04 Huddinge (SE)
(72) Inventor: Högberg, Marita, 141 04, Huddinge (SE); Johansson, Tommy, 141 04, Huddinge (SE); Dahlstedt, Emma, 140 04, Huddinge (SE); Smitt, Olof, 141 04, Huddinge (SE)
(74) Representative: Lundquist, Tomas

(57) **Abstract**

The invention provides novel pyrimidine derivatives of formula I, to methods of preparing such compounds, to pharmaceutical compositions containing such compounds, and to methods for using such compounds in treatment of diseases including cancer; 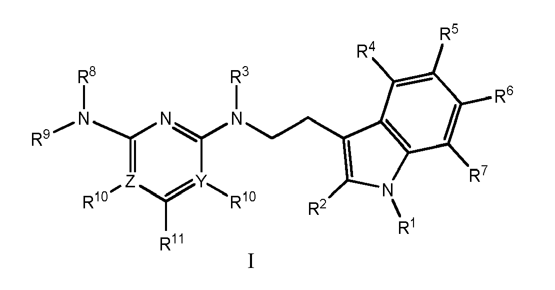 wherein R¹-R¹¹, Z, and Y are as defined in the specification.

## Description

### Field of Invention

This invention relates to novel pyrimidine derivatives, to methods of preparing such compounds, to pharmaceutical compositions containing such compounds, and to methods for using such compounds in treatment of diseases including cancer.

### Background of Invention

Cancer is a major and often fatal disease. Accordingly, the development of new therapies for cancer is an ongoing process of outmost importance. The majority of cancers are present as solid tumours, such as lung cancer, breast cancer, and prostate cancer, while others represent haematological and lymphoid malignancies, such as leukemias and lymphomas.

One important molecular target for the cancer chemotherapy is tubulin. The targeting drugs in this therapy interrupt microtubule spindle-mediated chromosome segregation, arrest the dividing tumor cells in mitosis and subsequently induce apoptosis. Existing drugs are targeting microtubules via two different mechanisms, e.g. molecules of the taxane class (that stabilize the tubulins) and several vinca alkaloids (destabilizers). The potency, efficacy, and widespread clinical use of these agents of natural origin in a variety of cancers, e.g. breast, ovarian, prostate, lung, leukemias, and lymphomas, stand testament to the importance of tubulin and its role in cancer growth. Derivatives and analogs of these plant compounds are constantly being isolated or synthesized to find more efficacious anticancer agents. For examples of novel tubulin polymerization inhibitors, see e.g. WO 2009/070645 and US 2010/0279410.

In the clinic cancer chemotherapy is used in attempts to cure or palliate the disease. In most cases this therapy is delivered in the form of combination chemotherapy, i.e. when two or more drugs having different modes of action are used together in order to optimise the effect on the cancer cells and to minimise side effects. The results obtained with chemotherapy vary according to tumour type. Some tumours are very sensitive and the treatment has a high probability of leading to beneficial treatment results including cure of the disease. Examples of this type of tumours are acute leukemias, malignant lymphomas, testicular cancer, chorion carcinomas, and Wilms tumour. Other types of cancer chemotherapy can result in effective palliation and prolonged survival. Examples of such tumours are breast cancer, colorectal cancer, ovarian cancer, small-cell lung cancer, bladder cancer, multiple myeloma, and chronic leukemias of both the lymphatic and myeloid type. Primary drug resistant tumours which respond poorly to classical chemotherapy include malignant glioma, melanoma, prostate cancer, sarcomas, and gastrointestinal tumours other than colorectal cancers (e.g. DeVita, Hellman, and Rosenberg: Cancer: Principles & Practice of Oncology, Eighth Edition 978-0-7817-7207-5).

During the recent decade much interest has been devoted to drugs directed to specific target molecules. Molecules regulating cell proliferation and death, such as Tyrosine Kinase Receptors (RTKs) for growth factors, are among targets for this type of therapeutic approach. Two classes of compounds targeting RTKs are currently used in clinical practice: monoclonal antibodies and tyrosine kinase inhibitors. The first approved targeted therapies were trastuzumab, a monoclonal antibody against HER2, for treatment of metastatic breast cancer, and imatinib, a small tyrosine kinase inhibitor targeting BCR-Abl, in Chronic Myeloid Leukemia. Despite good treatment results many of the treated patients have developed drug resistance, often due to the activation of alternative RTKs pathways. Currently there is a general idea that molecules interfering simultaneously with multiple RTKs might be more effective than single target agents. There are a few recently approved drugs, such as sorafenib and sunitinib, that apparently target multiple pathways and could serve as representatives of this new generation of anti-cancer drugs (e.g. Gossage and Eisen, Targeting multiple kinase pathways: a change in paradigm. Clin Cancer Res (2010) vol. 16(7) pp. 1973-8).

Certain pyrimidine compounds and their potential use in the treatment of cancer are disclosed in for example WO2003/030909, WO2003/059913, WO2003/063794, WO2004/056807, WO2004/056786, WO2006/133426, WO2007/085833, WO2008/128231, and WO2009/063240.

What is needed in the art are targeted drugs that work in a specific manner, being selective in eliminating subpopulations of cells involved in tumour survival and progression. The present invention provides novel pyrimidine compounds that have a surprisingly efficient antiproliferative activity. Hence, these novel compounds are useful in the treatment of proliferative diseases, such as cancer.

### Description of the invention

In a first aspect of the present invention, there is provided compounds of formula I or a pharmaceutically acceptable ester, amide, solvate or salt thereof, 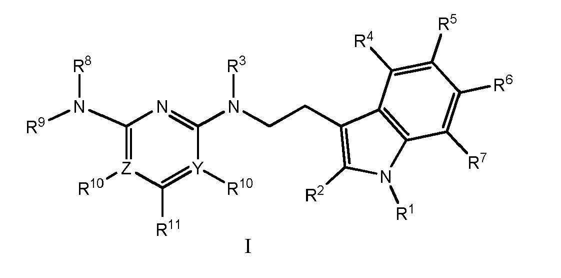 wherein
Z represents carbon or nitrogen;
Y represents carbon or nitrogen, wherein one of Z and Y represents nitrogen;
R¹, R³, and R⁸ are independently selected from hydrogen and (C₁-C₄)alkyl;
R² is selected from hydrogen, (C₁-C₄)alkyl, (C₁-C₄)alkyl-NH(C₁-C₄)alkyl, (C₁-C₄)alkyl-N[(C₁-C₄)alkyl]₂, and (CO)OH;
R⁴, R⁵, R⁶, and R⁷ are independently selected from hydrogen, halogen, hydroxy, amino, nitro, cyano, (C₁-C₄)alkyl, (C₁-C₄)alkyl-OH, (C₁-C₄)alkyl-NH₂, (C₁-C₄)alkyl-NH(C₁-C₄)alkyl, (C₁-C₄)alkyl-N[(C₁-C₄)alkyl]₂, (C₁-C₄)alkyl-NH(CO)(C₁-C₄)alkyl, (CO)OH, (CO)NH₂, (CO)NH(C₁-C₄)alkyl, (CO)N[(C₁-C₄)alkyl]₂, O(C₁-C₄)alkyl, O(C₁-C₄)alkyl(C₂-C₅)heterocyclyl, O(C₁-C₄)alkyl(C₂-C₅)heterocyclyl(C₁-C₄)alkyl, O(C₁-C₄)alkyl(CO)OH, O(C₁-C₄)alkyl(CO)NH(C₁-C₄)alkyl, O(C₁-C₄)alkyl(CO)N[(C₁-C₄)alkyl]₂, OCF₃, NH(C₁-C₄)alkyl, N[(C₁-C₄)alkyl]₂, NH(CO)(C₁-C₄)alkyl, NHSO₂(C₁-C₄)alkyl, N[(C₁-C₄)alkyl]SO₂(C₁-C₄)alkyl, SH, S(C₁-C₄)alkyl, SO₂NH₂, SO₂NH(C₁-C₄)alkyl, and SO₂N[(C₁-C₄)alkyl]₂;
R¹⁰ is selected from hydrogen, amino, and (C₁-C₄)alkyl when Z or Y is carbon;
R¹¹ is selected from hydrogen, amino, (C₁-C₄)alkyl, (C₁-C₄)alkyl(C₂-C₅)heterocyclyl, (C₁-C₄)alkyl(C₂-C₅)heterocyclyl(C₁-C₄)alkyl, (CO)OH, (CO)NH₂, (CO)NH(C₁-C₄)alkyl, (CO)N[(C₁-C₄)alkyl]₂, (CO)(C₁-C₄)alkyl, (C₂-C₅)heterocyclyl, (C₂-C₅)heterocyclyl(C₁-C₄)alkyl, NH(C₁-C₄)alkyl, N[(C₁-C₄)alkyl]₂, NH(CO)(C₁-C₄)alkyl, NHSO₂(C₁-C₄)alkyl, N[(C₁-C₄)alkyl]SO₂(C₁-C₄)alkyl, SO₂NH₂, SO₂NH(C₁-C₄)alkyl, and SO₂N[(C₁-C₄)alkyl]₂;
R⁹ represents 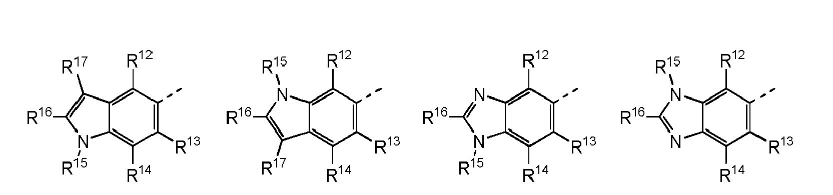 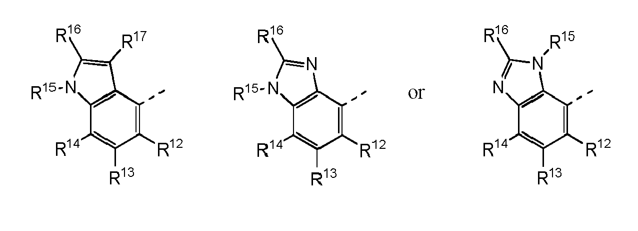 R¹², R¹³, and R¹⁴ are independently selected from hydrogen, halogen, hydroxy, (C₁-C₄)alkyl, O(C₁-C₄)alkyl, NH(C₁-C₄)alkyl, and N[(C₁-C₄)alkyl]₂;
R¹⁵ is selected from hydrogen and (C₁-C₄)alkyl; and
R¹⁶ and R¹⁷ are independently selected from hydrogen, halogen, (C₁-C₄)alkyl, (C₁-C₄)alkyl(C₂-C₅)heterocyclyl, (C₁-C₄)alkyl(C₂-C₅)heterocyclyl(C₁-C₄)alkyl, (C₁-C₄)alkyl(CO)OH, (C₁-C₄)alkyl(CO)NH₂, (C₁-C₄)alkyl(CO)NH(C₁-C₄)alkyl, (C₁-C₄)alkyl(CO)N[(C₁-C₄)alkyl]₂, (C₁-C₄)alkyl-OH, (C₁-C₄)alkyl-O(C₁-C₄)alkyl, (C₁-C₄)alkyl-NH(C₁-C₄)alkyl, (C₁-C₄)alkyl-N[(C₁-C₄)alkyl]₂, (C₁-C₄)alkyl-NH(CO)(C₁-C₄)alkyl, (CO)OH, (CO)NH₂, (CO)NH(C₁-C₄)alkyl, (CO)N[(C₁-C₄)alkyl]₂, (CO)(C₁-C₄)alkyl, (CO)(C₂-C₅)heterocyclyl, and (CO)(C₂-C₅)heterocyclyl)(C₁-C₄)alkyl.

In one embodiment of this aspect, R¹, R², R³, and R⁸ represent hydrogen.

In another embodiment of this aspect, R⁴, R⁵, R⁶, and R⁷ are independently selected from hydrogen, halogen, hydroxy, (C₁-C₄)alkyl, O(C₁-C₄)alkyl, O(C₁-C₄)alkyl(C₂-C₅)heterocyclyl, and OCF₃.

In another embodiment of this aspect, R¹⁰ is selected from hydrogen and (C₁-C₄)alkyl when Z or Y is carbon.

In another embodiment of this aspect, R¹⁰ is selected from hydrogen and methyl when Z or Y is carbon.

In another embodiment of this aspect, R¹¹ is selected from hydrogen, (C₁-C₄)alkyl, (CO)NH₂, and (C₂-C₅)heterocyclyl(C₁-C₄)alkyl.

In another embodiment of this aspect, R⁹ is selected from

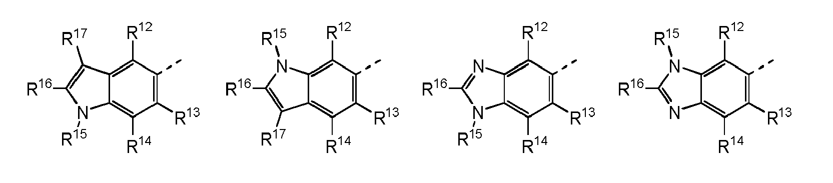

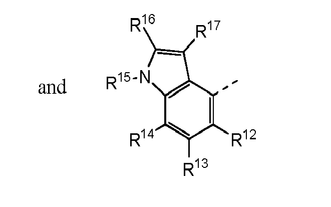

In another embodiment of this aspect, R¹², R¹³, and R¹⁴ represent hydrogen.

In another embodiment of this aspect, R¹⁵ is selected from hydrogen and methyl.

In another embodiment of this aspect, R¹⁶ and R¹⁷ are independently selected from hydrogen, (C₁-C₄)alkyl, (C₁-C₄)alkyl-OH, and (CO)OH.

In another embodiment of this aspect, R¹⁶ and R¹⁷ are independently selected from hydrogen, methyl, (C₁-C₄)alkyl-OH, and (CO)OH.

In another embodiment of this aspect, R¹⁶ is selected from hydrogen, methyl, (C₁-C₄)alkyl-OH, and (CO)OH.

In another embodiment of this aspect, R¹⁷ is selected from hydrogen and methyl.

In another embodiment of this aspect, Z represents carbon and Y represents nitrogen.

In another embodiment of this aspect, Y represents carbon and Z represents nitrogen.

In another embodiment of this aspect,
Z represents carbon and Y represents nitrogen;
R¹, R², R³, R⁸, R¹², R¹³, and R¹⁴ represent hydrogen;
R⁴, R⁵, R⁶, and R⁷ are independently selected from hydrogen, halogen, hydroxy, (C₁-C₄)alkyl, O(C₁-C₄)alkyl, O(C₁-C₄)alkyl(C₂-C₅)heterocyclyl, and OCF₃;
R¹⁰ is selected from hydrogen and (C₁-C₄)alkyl when Z or Y is carbon;
R¹¹ is selected from hydrogen, (C₁-C₄)alkyl, (CO)NH₂, and (C₂-C₅)heterocyclyl(C₁-C₄)alkyl;
R⁹ is selected from 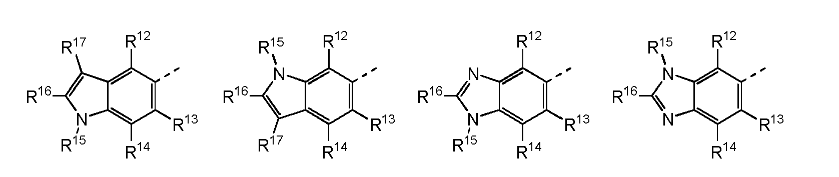 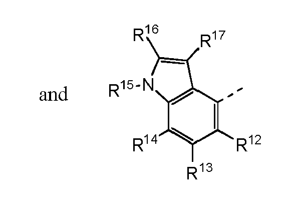 R¹⁵ is selected from hydrogen and methyl;
and R¹⁶ and R¹⁷ are independently selected from hydrogen, (C₁-C₄)alkyl, (C₁-C₄)alkyl-OH, and (CO)OH.

In another embodiment of this aspect,
R⁴ represents hydrogen;
R⁵ is selected from halogen, methyl, O(C₁-C₂)alkyl, and OCF₃;
R⁶ and R⁷ are independently selected from hydrogen, methyl, and methoxy;
R¹⁰ is selected from hydrogen and methyl when Z or Y is carbon;
R¹¹ is selected from hydrogen, methyl, and (CO)NH₂;
R⁹ is selected from 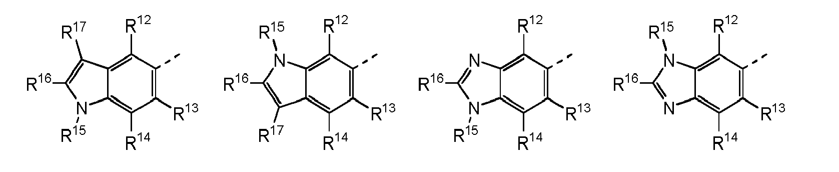 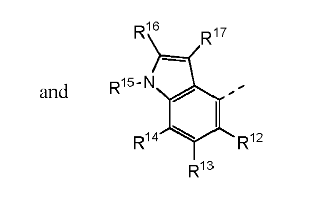 R¹⁵ is selected from hydrogen and methyl;
and R¹⁶ and R¹⁷ are independently selected from hydrogen, methyl, and hydroxymethyl.

In another embodiment of this aspect, R¹⁶ represents methyl.

In another embodiment of this aspect, R⁵ is selected from methoxy, methyl, and fluoro.

In another embodiment of this aspect, R⁵ is selected from methoxy, and methyl.

In another embodiment of this aspect, R¹¹ is selected from hydrogen, methyl, and (CO)NH₂.

In another embodiment of this aspect, R⁹ is selected from 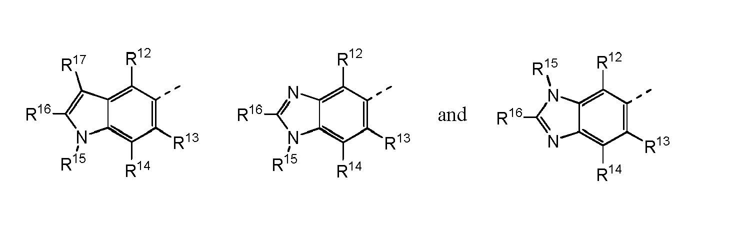 wherein R¹⁵ and R¹⁷ are independently selected from hydrogen and methyl; and R¹⁶ is selected from hydrogen, methyl, hydroxymethyl, and (CO)OH.

In another aspect of the invention, there is provided a compound of formula I, said compound being selected from:
*N²-*[2-(1*H*-indol-3-yl)ethyl]-*N⁴-*(1*H*-indol-5-yl)pyrimidine-2,4-diamine;
*N*⁴*-*(1*H-indol-5-*yl)*-N*²*-*[2-(5-methoxy-1*H-*indol-3-yl)ethyl]pyrimidine-2,4-diamine;
*N²*-[2-(1*H-*indol-3-yl)ethyl]-*N⁴*-(2-methyl-1*H-*indol-5-yl)pyrimidine-2,4-diamine;
*N²*-[2-(5-methoxy-1*H*-indol-3-yl)ethyl]-*N⁴*-(2-methyl-1*H-*indol-5-yl)pyrimidine-2,4-diamine;
*N²*-[2-(5-ethoxy-1*H-*indol-3-yl)ethyl]-*N⁴*-(2-methyl-1*H-*indol-5-yl)pyrimidine-2,4-diamine;
*N*⁴-(2-methyl-1*H-*indol-5-yl)-*N*²-{2-[5-(2-morpholinoethoxy)-1*H*-indol-3-yl]ethyl}pyrimidine-2,4-diamine;
*N*⁴-(2-methyl-1*H-*indol-5-yl)-*N*²-{2-[5-(trifluoromethoxy)-1*H*-indol-3-yl]ethyl}pyrimidine-2,4-diamine;
3-{2-[4-(2-methyl-1*H*-indol-5-ylamino)pyrimidin-2-ylamino]ethyl}-1*H*-indol-5-ol; *N*²-[2-(5-methyl-1*H*-indol-3-yl)ethyl]-*N*⁴-(2-methyl-1*H*-indol-5-yl)pyrimidine-2,4-diamine;
*N*²-[2-(5-fluoro-1*H*-indol-3-yl)ethyl]-*N*⁴-(2-methyl-1*H*-indol-5-yl)pyrimidine-2,4-diamine;
*N*²-[2-(6-methoxy-1*H*-indol-3-yl)ethyl]-*N*⁴-(2-methyl-1*H*-indol-5-yl)pyrimidine-2,4-diamine;
*N*²-[2-(7-methoxy-1*H*-indol-3-yl)ethyl]-*N*⁴-(2-methyl-1*H*-indol-5-yl)pyrimidine-2,4-diamine;
*N*⁴-(1,2-dimethyl-1*H*-indol-5-yl)-*N*²-[2-(5-methoxy-1*H*-indol-3-yl)ethyl]pyrimidine-2,4-diamine;
*N*⁴-(2,3-dimethyl-1*H*-indol-5-yl)-*N*²-[2-(5-methoxy-1*H*-indol-3-yl)ethyl]pyrimidine-2,4-diamine;
(5-{2-[2-(5-methoxy-1*H*-indol-3-yl)ethylamino]pyrimidin-4-ylamino}-1*H*-indol-2-yl)methanol;
methyl 5-{2-[2-(5-methoxy-1*H*-indol-3-yl)ethylamino]pyrimidin-4-ylamino}-1*H-*indole-2-carboxylate;
2-hydroxyethyl 5-{2-[2-(5-methoxy-1*H-*indol-3-yl)ethylamino]pyrimidin-4-ylamino}-1*H*-indole-2-carboxylate;
*N*⁴-(1*H*-benzo[*d*]imidazol-5-yl)-*N*²-[2-(5-methoxy-1*H*-indol-3-yl)ethyl]pyrimidine-2,4-diamine;
*N*²-[2-(5-methoxy-1*H*-indol-3-yl)ethyl]-*N*⁴-(2-methyl-1*H*-benzo[*d*]imidazol-5-yl)pyrimidine-2,4-diamine;
*N*⁴-(1*H*-indol-6-yl)-*N*²-[2-(5-methoxy-1*H*-indol-3-yl)ethyl]pyrimidine-2,4-diamine;
*N*⁴-(1*H*-indol-4-yl)-*N*²-[2-(5-methoxy-1*H*-indol-3-yl)ethyl]pyrimidine-2,4-diamine;
*N*⁴-[2-(1*H*-indol-3-yl)ethyl]-*N*²-(2-methyl-1*H*-indol-5-yl)pyrimidine-2,4-diamine;
*N*⁴-[2-(5-methoxy-1*H*-indol-3-yl)ethyl]-*N*²-(2-methyl-1*H*-indol-5-yl)pyrimidine-2,4-diamine;
*N*⁴-[2-(1*H*-indol-3-yl)ethyl]-*N*²-(1*H*-indol-6-yl)pyrimidine-2,4-diamine;
*N*⁴-[2-(1*H*-indol-3-yl)ethyl]-*N*²-(1*H*-indol-4-yl)pyrimidine-2,4-diamine;
*N*²-[2-(1*H*-indol-3-yl)ethyl]-6-methyl-*N*⁴-(2-methyl-1*H*-indol-5-yl)pyrimidine-2,4-diamine;
*N*²-[2-(5-methoxy-1*H*-indol-3-yl)ethyl]-6-methyl-*N*⁴-(2-methyl-1*H*-indol-5-yl)pyrimidine-2,4-diamine;
2-[2-(5-methoxy-1*H*-indol-3-yl)ethylamino]-6-(2-methyl-1*H*-indol-5-ylamino)pyrimidine-4-carboxamide;
*N*²-[2-(5-methoxy-1*H*-indol-3-yl)ethyl]-5-methyl-*N*⁴-(2-methyl-1*H*-indol-5-yl)pyrimidine-2,4-diamine; and
*N*²-[2-(5-methoxy-1*H*-indol-3-yl)ethyl]-*N*⁴-(2-methyl-1*H*-indol-5-yl)-6-(4-methylpiperazin-1-yl)pyrimidine-2,4-diamine.

In another aspect of the invention, there is provided a compound of formula I, for use in therapy.

In another aspect of the invention, there is provided a compound of formula I, for use in treatment of cancer.

In another aspect of the invention, there is provided use of a compound of formula I, in the manufacture of a medicament and pharmaceutical compositions for treatment of cancer.

In another aspect of the invention, there is provided a pharmaceutical composition comprising a compound of formula I, together with pharmaceutically acceptable diluents and carriers.

In another aspect of the invention, there is provided a method for treatment of cancer, which comprises administering to a subject in need thereof, a therapeutically effective amount of a compound of formula I.

In another aspect of the invention, there is provided a method for treatment of cancer, which comprises administering to a subject in need thereof, a therapeutically effective amount of a compound of formula I, in combination with another compound of formula I, in combination with radiation therapy, or in combination with another anticancer agent selected from alkylating agents, antimetabolites, anticancer camptothecin derivatives, plant-derived anticancer agents, antibiotics, enzymes, platinum coordination complexes, tubulin inhibitors, tyrosine kinase inhibitors, hormones, hormone antagonists, monoclonal antibodies, interferons, and biological response modifiers.

Further compounds related to the invention include, but are not limited to, the following compounds:
*N*²-[2-(5-methoxy-1-methyl-1*H*-indol-3-yl)ethyl]-*N*⁴-(2-methyl-1*H*-indol-5-yl)pyrimidine-2,4-diamine;
*N*²-[2-(5-methoxy-1*H*-indol-3-yl)ethyl]-*N*²-methyl-*N*⁴-(2-methyl-1*H*-indol-5-yl)pyrimidine-2,4-diamine;
*N*²-[2-(5-methoxy-1*H*-indol-3-yl)ethyl]-*N*⁴-methyl-*N*⁴-(2-methyl-1*H*-indol-5-yl)pyrimidine-2,4-diamine;
*N*²-[2-(5-methoxy-1-methyl-1*H*-indol-3-yl)ethyl]-*N*²-methyl-*N*⁴-(2-methyl-1*H*-indol-5-yl)pyrimidine-2,4-diamine;
*N*²-[2-(5-methoxy-1-methyl-1*H*-indol-3-yl)ethyl]-*N*⁴-methyl-*N*⁴-(2-methyl-1*H*-indol-5-yl)pyrimidine-2,4-diamine;
*N*4-(1,2-dimethyl-1*H*-indol-5-yl)-*N*²-[2-(5-methoxy-1-methyl-1*H*-indol-3-yl)ethyl]pyrimidine-2,4-diamine;
*N*²-[2-(5-methoxy-1*H*-indol-3-yl)ethyl]-*N*²,*N*⁴-dimethyl-*N*⁴-(2-methyl-1*H*-indol-5-yl)pyrimidine-2,4-diamine;
*N*⁴-(1,2-dimethyl-1*H*-indol-5-yl)-*N*²-[2-(5-methoxy-1*H*-indol-3-yl)ethyl]-*N*²-methylpyrimidine-2,4-diamine;
*N*⁴-(1,2-dimethyl-1*H*-indol-5-yl)-*N*²-[2-(5-methoxy-1*H*-indol-3-yl)ethyl]-*N*⁴-methylpyrimidine-2,4-diamine;
*N*²-[2-(5-methoxy-1-methyl-1*H*-indol-3-yl)ethyl]-*N*²,*N*⁴-dimethyl-*N*⁴-(2-methyl-1*H-*indol-5-yl)pyrimidine-2,4-diamine;
*N*⁴-(1,2-dimethyl-1*H*-indol-5-yl)-*N*²-[2-(5-methoxy-1-methyl-1*H*-indol-3-yl)ethyl]-*N*²-methylpyrimidine-2,4-diamine;
*N*⁴-(1,2-dimethyl-1*H*-indol-5-yl)-*N*²-[2-(5-methoxy-1-methyl-1*H*-indol-3-yl)ethyl]-*N*⁴-methylpyrimidine-2,4-diamine;
*N*⁴-(1,2-dimethyl-1*H*-indol-5-yl)-*N*²-[2-(5-methoxy-1*H*-indol-3-yl)ethyl]-*N*²,*N*⁴-dimethylpyrimidine-2,4-diamine;
*N*⁴-(1,2-dimethyl-1*H*-indol-5-yl)-*N*²-[2-(5-methoxy-1-methyl-1*H*-indol-3-yl)ethyl]-*N*²,*N*⁴-dimethylpyrimidine-2,4-diamine;
*N*⁴-(1,2-dimethyl-1*H*-benzo[*d*]imidazol-5-yl)-*N*²-[2-(5-methoxy-1*H*-indol-3-yl)ethyl]pyrimidine-2,4-diamine;
*N*⁴-(1,2-dimethyl-1*H*-benzo[*d*]imidazol-6-yl)-*N*²-[2-(5-methoxy-1*H*-indol-3-yl)ethyl]pyrimidine-2,4-diamine;
*N*²-[2-(5-methoxy-1*H*-indol-3-yl)ethyl]-*N*⁴-(1-methyl-1*H*-indol-6-yl)pyrimidine-2,4-diamine;
*N*²-[2-(5-methoxy-1*H*-indol-3-yl)ethyl]-*N*⁴-(1-methyl-1*H*-indol-4-yl)pyrimidine-2,4-diamine;
*N*²-[2-(5-isopropoxy-1*H*-indol-3-yl)ethyl)]-*N*⁴-(2-methyl-1*H*-indol-5-yl)pyrimidine-2,4-diamine;
*N*²-[2-(5,6-dimethoxy-1*H*-indol-3-yl)ethyl]-*N*⁴-(2-methyl-1*H*-indol-5-yl)pyrimidine-2,4-diamine;
*N*²-[2-(5,7-dimethoxy-1*H*-indol-3-yl)ethyl]-*N*⁴-(2-methyl-1*H*-indol-5-yl)pyrimidine-2,4-diamine;
*N*²-[2-(5,7-dimethoxy-2-methyl-1*H*-indol-3-yl)ethyl]-*N*⁴-(2-methyl-1*H*-indol-5-yl)pyrimidine-2,4-diamine;
*N*²-[2-(4,7-dimethoxy-1*H*-indol-3-yl)ethyl]-*N*⁴-(2-methyl-1*H*-indol-5-yl)pyrimidine-2,4-diamine;
*N*⁴-(2-methyl-1*H*-indol-5-yl)-*N*²-[2-(5,6,7-trimethoxy-1*H*-indol-3-yl)ethyl]pyrimidine-2,4-diamine;
*N*²-[2-(4-methoxy-1*H*-indol-3-yl)ethyl]-*N*⁴-(2-methyl-1*H*-indol-5-yl)pyrimidine-2,4-diamine;
5-methoxy-3-{2-[4-(2-methyl-1*H*-indol-5-ylamino)pyrimidin-2-ylamino]ethyl}-1*H-*indole-2-carboxylic acid;
*N*²-[2-(4-methyl-1*H*-indol-3-yl)ethyl]-*N*⁴-(2-methyl-1*H*-indol-5-yl)pyrimidine-2,4-diamine;
*N*²-[2-(6-methyl-1*H*-indol-3-yl)ethyl]-*N*⁴-(2-methyl-1*H*-indol-5-yl)pyrimidine-2,4-diamine;
*N*²-[2-(7-methyl-1*H*-indol-3-yl)ethyl]-*N*⁴-(2-methyl-1*H*-indol-5-yl)pyrimidine-2,4-diamine;
*N*²-[2-(2-methyl-1*H*-indol-3-yl)ethyl]-*N*⁴-(2-methyl-1*H*-indol-5-yl)pyrimidine-2,4-diamine;
*N*²-[2-(6-fluoro-1*H*-indol-3-yl)ethyl]-*N*⁴-(2-methyl-1*H*-indol-5-yl)pyrimidine-2,4-diamine;
*N*⁴-(2-methyl-1*H*-indol-5-yl)-*N*²-{2-[5-(methylthio)-1*H*-indol-3-yl]-ethyl}pyrimidine-2,4-diamine;
3-{2-[4-(2-methyl-1*H*-indol-5-ylamino)pyrimidin-2-ylamino]ethyl}-1*H*-indole-2-carboxylic acid;
(5-{2-[2-(5-methoxy-1*H*-indol-3-yl)ethylamino]pyrimidin-4-ylamino}-2-methyl-1*H-*indol-3-yl)methanol;
*N*⁴-{3-[(dimethylamino)methyl]-2-methyl-1*H*-indol-5-yl}-*N*²-[2-(5-methoxy-1*H-*indol-3-yl)ethyl]pyrimidine-2,4-diamine;
2-(5-{2-[2-(5-methoxy-1*H*-indol-3-yl)ethylamino]pyrimidin-4-ylamino}-2-methyl-1*H*-indol-3-yl)acetic acid;
2-(5-{2-[2-(5-methoxy-1*H*-indol-3-yl)ethylamino]pyrimidin-4-ylamino}-2-methyl-1*H*-indol-3-yl)acetamide;
*N*⁴-(4-fluoro-2-methyl-1*H*-indol-5-yl)-*N*²-[2-(5-methoxy-1*H*-indol-3-yl)ethyl]pyrimidine-2,4-diamine;
5-{2-[2-(5-methoxy-1*H*-indol-3-yl)ethylamino]pyrimidin-4-ylamino}-1*H*-indole-2-carboxylic acid;
5-{2-[2-(5-methoxy-1*H*-indol-3-yl)ethylamino]pyrimidin-4-ylamino}-1*H*-indole-2-carboxamide;
(5-{2-[2-(5-methoxy-1*H*-indol-3-yl)ethylamino]pyrimidin-4-ylamino}-1*H*-indol-2-yl)(pyrrolidin-1-yl)methanone;
*N*²-[2-(5-methoxy-1*H-*indol-3-yl)ethyl]-*N*⁴-(3-methyl-1*H*-indol-5-yl)pyrimidine-2,4-diamine;
*N*-[2-(5-{2-[2-(5-methoxy-1*H*-indol-3-yl)ethylammo]pyrimidin-4-ylamino}-1*H-*indol-3-yl)ethyl]acetamide;
*N*⁴-{3-[(dimethylamino)methyl]-1*H*-indol-5-yl}-*N*²-[2-(5-methoxy-1*H*-indol-3-yl)ethyl]pyrimidine-2,4-diamine;
5-{2-[2-(5-methoxy-1*H*-indol-3-yl)ethylamino]pyrimidin-4-ylamino}-1*H*-indole-3-carboxylic acid;
5-{2-[2-(5-methoxy-1*H*-indol-3-yl)ethylamino]pyrimidin-4-ylamino}-1*H*-indole-3-carboxamide;
*N*²-[2-(5-methoxy-1*H*-indol-3-yl)ethyl]-*N*⁴-(7-methyl-1*H*-indol-5-yl)pyrimidine-2,4-diamine;
*N*²-[2-(5-methoxy-1*H*-indol-3-yl)ethyl]-*N*⁴-(4-methoxy-1*H*-indol-5-yl)pyrimidine-2,4-diamine;
*N*⁴-(2,4-dimethyl-1*H-*benzo[*d*]imidazol-5-yl)-*N*²-[2-(5-methoxy-1*H*-indol-3-yl)ethyl]pyrimidine-2,4-diamine;
*N*²-[2-(5-methoxy-1*H*-indol-3-yl)ethyl]-*N*⁴-(6-methoxy-2-methyl-1*H-*benzo[*d*]imidazol-5-yl)pyrimidine-2,4-diamine;
5-{2-[2-(5-methoxy-1*H*-indol-3-yl)ethylamino]pyrimidin-4-ylamino}-2-methyl-1*H-*benzo[*d*]imidazol-6-l;
5-{2-[2-(5-methoxy-1*H*-indol-3-yl)ethylamino]pyrimidin-4-ylamino}-2-methyl-1*H-*benzo[*d*]imidazol-7-ol;
*N*⁴-(2,6-dimethyl-1*H*-benzo[*d*]imidazol-5-yl)-*N*²-[2-(5-methoxy-1*H*-indol-3-yl)ethyl]pyrimidine-2,4-diamine;
*N*⁴-{2-[(dimethylamino)methyl]-1*H*-benzo[*d*]imidazol-5-yl}-*N*²-[2-(5-methoxy-1*H-*indol-3-yl)ethyl]pyrimidine-2,4-diamine;
*N*²-[2-(5-methoxy-1*H*-indol-3-yl)ethyl]-*N*⁴-[2-(morpholinomethyl)-1*H-*benzo[*d*]imidazol-5-yl]pyrimidine-2,4-diamine;
2-(5-{2-[2-(5-methoxy-1*H*-indol-3-yl)ethylamino]pyrimidin-4-ylamino}-1*H-*benzo[*d*]imidazol-2-yl)ethanol;
*N*²-[2-(5-methoxy-1*H*-indol-3-yl)ethyl]-*N*⁴-[2-(methoxymethyl)-1*H-*benzo[*d*]imidazol-5-yl]pyrimidine-2,4-diamine;
*N*²-[2-(5-methoxy-1*H*-indol-3-yl)ethyl]-*N*⁴-(2-methyl-1*H*-indol-6-yl)pyrimidine-2,4-diamine;
*N*²-[2-(5-methoxy-1*H*-indol-3-yl)ethyl]-*N*⁴-(2-methyl-1*H*-indol-4-yl)pyrimidine-2,4-diamine;
*N⁴*-(1*H*-benzo[*d*]imidazol-4-yl)-*N*²-[2-(5-methoxy-1*H*-indol-3-yl)ethyl]pyrimidine-2,4-diamine;
*N*²-[2-(5-methoxy-1*H*-indol-3-yl)ethyl]-*N*⁴-(2-methyl-1*H*-benzo[*d*]imidazol-4-yl)pyrimidine-2,4-diamine;
2-[2-(5-methoxy-1*H*-indol-3-yl)ethylamino]-*N*-methyl-6-(2-methyl-1*H*-indol-5-ylamino)pyrimidine-4-carboxamide;
2-[2-(5-methoxy-1*H*-indol-3-yl)ethylamino]-*N*,*N*-dimethyl-6-(2-methyl-1*H*-indol-5-ylamino)pyrimidine-4-carboxamide;
2-[2-(5-methoxy-1*H*-indol-3-yl)ethylamino]-6-(2-methyl-1*H*-indol-5-ylamino)pyrimidine-4-carboxylic acid;
*N*-{2-[2-(5-methoxy-1*H*-indol-3-yl)ethylamino]-6-(2-methyl-1*H*-indol-5-ylamino)pyrimidin-4-yl} acetamide;
2-[2-(5-methoxy-1*H*-indol-3-yl)ethylamino]-6-(2-methyl-1*H*-indol-5-ylamino)pyrimidine-4-sulfonamide;
2-[2-(5-methoxy-1*H*-indol-3-yl)ethylamino]-*N*,*N*-dimethyl-6-(2-methyl-1*H*-indol-5-ylamino)pyrimidine-4-sulfonamide;
*N*²-[2-(5-methoxy-1*H*-indol-3-yl)ethyl]-*N*⁴-(2-methyl-1*H*-indol-5-yl)pyrimidine-2,4,5-triamine;
*N*⁴-(1,2-dimethyl-1*H*-indol-5-yl)-*N*²-[2-(5-methoxy-1*H*-indol-3-yl)ethyl]-6-methylpyrimidine-2,4-diamine;
*N*²-[2-(5-ethoxy-1*H*-indol-3-yl)ethyl]-6-methyl-*N*⁴-(2-methyl-1*H*-indol-5-yl)pyrimidine-2,4-diamine;
6-methyl-*N*⁴-(2-methyl-1*H*-indol-5-yl)-*N*²-{2-[5-(trifluoromethoxy)-1*H*-indol-3-yl]ethyl}pyrimidine-2,4-diamine;
6-methyl-*N*²-[2-(5-methyl-1*H*-indol-3-yl)ethyl]-*N*⁴-(2-methyl-1*H*-indol-5-yl)pyrimidine-2,4-diamine;
*N*²-[2-(5-fluoro-1*H*-indol-3-yl)ethyl]-6-methyl-*N*⁴-(2-methyl-1*H*-indol-5-yl)pyrimidine-2,4-diamine;
*N*²-[2-(6-methoxy-1*H*-indol-3-yl)ethyl]-6-methyl-*N*⁴-(2-methyl-1*H*-indol-5-yl)pyrimidine-2,4-diamine;
*N*²-[2-(7-methoxy-1*H*-indol-3-yl)ethyl]-6-methyl-*N*⁴-(2-methyl-1*H*-indol-5-yl)pyrimidine-2,4-diamine;
*N*⁴-(1*H*-indol-5-yl)-*N*²-[2-(5-methoxy-1*H*-indol-3-yl)ethyl]-6-methylpyrimidine-2,4-diamine;
*N*⁴-(2,3-dimethyl-1*H*-indol-5-yl)-*N*²-[2-(5-methoxy-1*H*-indol-3-yl)ethyl]-6-methylpyrimidine-2,4-diamine;
(5-{2-[2-(5-methoxy-1*H*-indol-3-yl)ethylamino]-6-methylpyrimidin-4-ylamino}-1*H-*indol-2-yl)methanol;
*N*²-[2-(5-methoxy-1*H*-indol-3-yl)ethyl]-6-methyl-*N*⁴-(2-methyl-1*H* benzo[*d*]imidazol-5-yl)pyrimidine-2,4-diamine;
*N*⁴-(1*H*-indol-6-yl)-*N*²-[2-(5-methoxy-1*H*-indol-3-yl)ethyl]-6-methylpyrimidine-2,4-diamine;
*N*⁴-(1*H*-indol-4-yl)-*N*²-[2-(5-methoxy-1*H*-indol-3-yl)ethyl]-6-methylpyrimidine-2,4-diamine;
6-(1,2-dimethyl-1*H*-indol-5-ylamino)-2-[2-(5-methoxy-1*H*-ndol-3-yl)ethylamino]pyrimidine-4-carboxamide;
2-[2-(5-ethoxy-1*H*-indol-3-yl)ethylamino]-6-(2-methyl-1*H*-indol-5-ylamino)pyrimidine-4-carboxamide;
6-(2-methyl-1*H*-indol-5-ylamino)-2-{2-[5-(trifluoromethoxy)-1*H*-indol-3-yl]ethylamino}pyrimidine-4-carboxamide;
2-[2-(5-methyl-1*H*-indol-3-yl)ethylamino]-6-(2-methyl-1*H*-indol-5-ylamino)pyrimidine-4-carboxamide;
2-[2-(5-fluoro-1*H*-indol-3-yl)ethylamino]-6-(2-methyl-1*H*-indol-5-ylamino)pyrimidine-4-carboxamide;
2-[2-(6-methoxy-1*H*-indol-3-yl)ethylamino]-6-(2-methyl-1*H*-indol-5-ylamino)pyrimidine-4-carboxamide;
2-[2-(7-methoxy-1*H*-indol-3-yl)ethylamino]-6-(2-methyl-1*H*-indol-5-ylamino)pyrimidine-4-carboxamide;
6-(1*H*-indol-5-ylamino)-2-[2-(5-methoxy-1*H*-indol-3-yl)ethylamino]pyrimidine-4-carboxamide;
6-(2,3-dimethyl-1*H*-indol-5-ylamino)-2-[2-(5-methoxy-1*H*-indol-3-yl)ethylamino]pyrimidine-4-carboxamide;
6-[2-(hydroxymethyl)-1*H*-indol-5-ylamino]-2-[2-(5-methoxy-1*H*-indol-3-yl)ethylamino]pyrimidine-4-carboxamide;
2-[2-(5-methoxy-1*H*-indol-3-yl)ethylamino]-6-(2-methyl-1*H*-benzo[*d*]imidazol-5-ylamino)pyrimidine-4-carboxamide;
6-(1*H*-indol-6-ylamino)-2-[2-(5-methoxy-1*H*-indol-3-yl)ethylamino]pyrimidine-4-carboxamide;
6-(1*H*-indol-4-ylamino)-2-[2-(5-methoxy-1*H*-indol-3-yl)ethylamino]pyrimidine-4-carboxamide;
*N*⁴-(1,2-dimethyl-1*H*-indol-5-yl)-*N*²-[2-(5-methoxy-1*H*-indol-3-yl)ethyl]-5-methylpyrimidine-2,4-diamine;
*N*²-[2-(5-ethoxy-1*H*-indol-3-yl)ethyl]-5-methyl-*N*⁴-(2-methyl-1*H*-indol-5-yl)pyrimidine-2,4-diamine;
5-methyl-*N*⁴-(2-methyl-1*H*-indol-5-yl)-*N*²-{2-[5-(trifluoromethoxy)-1*H*-indol-3-yl]ethyl}pyrimidine-2,4-diamine;
5-methyl-*N*2-[2-(5-methyl-1*H*-indol-3-yl)ethyl]-*N*4-(2-methyl-1*H*-indol-5-yl)pyrimidine-2,4-diamine;
*N*²-[2-(5-fluoro-1*H*-indol-3-yl)ethyl]-5-methyl-*N*⁴-(2-methyl-1*H*-indol-5-yl)pyrimidine-2,4-diamine;
*N*²-[2-(6-methoxy-1*H*-indol-3-yl)ethyl]-5-methyl-*N*⁴-(2-methyl-1*H*-indol-5-yl)pyrimidine-2,4-diamine;
*N*²-[2-(7-methoxy-1*H*-indol-3-yl)ethyl]-5-methyl-*N*⁴-(2-methyl-1*H*-indol-5-yl)pyrimidine-2,4-diamine;
*N*⁴-(1*H*-indol-5-yl)-*N*²-[2-(5-methoxy-1*H*-indol-3-yl)ethyl]-5-methylpyrimidine-2,4-diamine;
*N*⁴-(2,3-dimethyl-1*H*-indol-5-yl)-*N*²-[2-(5-methoxy-1*H*-indol-3-yl)ethyl]-5-methylpyrimidine-2,4-diamine;
(5-{2-[2-(5-methoxy-1*H*-indol-3-yl)ethylamino]-5-methylpyrimidin-4-ylamino}-1*H-*indol-2-yl)methanol;
*N*²-[2-(5-methoxy-1*H*-indol-3-yl)ethyl]-5-methyl-*N*⁴-(2-methyl-1*H-*benzo[*d*]imidazol-5-yl)pyrimidine-2,4-diamine;
*N*⁴-(1*H*-indol-6-yl)-*N*²-[2-(5-methoxy-1*H*-indol-3-yl)ethyl]-5-methylpyrimidine-2,4-diamine;
*N*⁴-(1*H*-indol-4-yl)-*N*²-[2-(5-methoxy-1*H*-indol-3-yl)ethyl]-5-methylpyrimidine-2,4-diamine;
*N*²-[2-(5-ethoxy-1*H*-indol-3-yl)ethyl]-*N*⁴-(1*H*-indol-5-yl)pyrimidine-2,4-diamine;
*N*⁴-(1*H*-indol-5-yl)-*N*²-{2-[5-(trifluoromethoxy)-1*H*-indol-3-yl]ethyl}pyrimidine-2,4-diamine;
*N*⁴-(1*H*-indol-5-yl)-*N*²-[2-(5-methyl-1*H*-indol-3-yl)ethyl]pyrimidine-2,4-diamine;
*N*²-[2-(5-fluoro-1*H*-indol-3-yl)ethyl]-*N*⁴-(1*H*-indol-5-yl)pyrimidine-2,4-diamine;
*N*⁴-(1*H*-indol-5-yl)-*N*²-[2-(6-methoxy-1*H*-indol-3-yl)ethyl]pyrimidine-2,4-diamine;
*N*⁴-(1*H*-indol-5-yl)-*N*²-[2-(7-methoxy-1*H*-indol-3-yl)ethyl]pyrimidine-2,4-diamine;
*N*⁴-(2,3-dimethyl-1*H*-indol-5-yl)-*N*²-[2-(5-ethoxy-1*H*-indol-3-yl)ethyl]pyrimidine-2,4-diamine;
*N*⁴-(2,3-dimethyl-1*H*-indol-5-yl)-*N*²-{2-[5-(trifluoromethoxy)-1*H*-indol-3-yl]ethyl}pyrimidine-2,4-diamine;
*N*⁴-(2,3-dimethyl-1*H*-indol-5-yl)-*N*²-[2-(5-methyl-1*H*-indol-3-yl)ethyl]pyrimidine-2,4-diamine;
*N*⁴-(2,3-dimethyl-1*H*-indol-5-yl)-*N*²-[2-(5-fluoro-1*H*-indol-3-yl)ethyl]pyrimidine-2,4-diamine;
*N*⁴-(2,3-dimethyl-1*H*-indol-5-yl)-*N*²-[2-(6-methoxy-1*H*-indol-3-yl)ethyl]pyrimidine-2,4-diamine;
*N*⁴-(2,3-dimethyl-1*H*-indol-5-yl)-*N*²-[2-(7-methoxy-1*H*-indol-3-yl)ethyl]pyrimidine-2,4-diamine;
(5-{2-[2-(5-ethoxy-1*H*-indol-3-yl)ethylamino]pyrimidin-4-ylamino}-1*H*-indol-2-yl)methanol;
[5-(2-{2-[5-(trifluoromethoxy)-1*H*-indol-3-yl]ethylamino}pyrimidin-4-ylamino)-1*H-*indol-2-yl]methanol;
(5-{2-[2-(5-methyl-1*H*-indol-3-yl)ethylamino]pyrimidin-4-ylamino}-1*H*-indol-2-yl)methanol;
(5-{2-[2-(5-fluoro-1*H*-indol-3-yl)ethylamino]pyrimidin-4-ylamino}-1*H*-indol-2-yl)methanol;
(5-{2-[2-(6-methoxy-1*H*-indol-3-yl)ethylamino]pyrimidin-4-ylamino}-1*H*-indol-2-yl)methanol;
(5-{2-[2-(7-methoxy-1*H*-indol-3-yl)ethylamino]pyrimidin-4-ylamino}-1*H*-indol-2-yl)methanol;
*N*²-[2-(5-ethoxy-1*H*-indol-3-yl)ethyl]-*N*⁴-(2-methyl-1*H*-benzo[*d*]imidazol-5-yl)pyrimidine-2,4-diamine;
*N*⁴-(2-methyl-1*H*-benzo[*d*]imidazol-5-yl)-*N*²-{2-[5-(trifluoromethoxy)-1*H*-indol-3-yl]ethyl}pyrimidine-2,4-diamine;
*N*⁴-(2-methyl-1*H*-benzo[*d*]imidazol-5-yl)-*N*²-[2-(5-methyl-1*H*-indol-3-yl)ethyl]pyrimidine-2,4-diamine;
*N*²-[2-(5-fluoro-1*H*-indol-3-yl)ethyl]-*N*⁴-(2-methyl-1*H*-benzo[*d*]imidazol-5-yl)pyrimidine-2,4-diamine;
*N*²-[2-(6-methoxy-1*H*-indol-3-yl)ethyl]-*N*⁴-(2-methyl-1*H*-benzo[*d*]imidazol-5-yl)pyrimidine-2,4-diamine;
*N*²-[2-(7-methoxy-1*H*-indol-3-yl)ethyl]-*N*⁴-(2-methyl-1*H*-benzo[*d*]imidazol-5-yl)pyrimidine-2,4-diamine; and
a pharmaceutically acceptable ester, amide, solvate or salt thereof.

In the list above and in the Examples, the compound names were generated in accordance with IUPAC by ChemBioDraw Ultra version 11.0.

Depending upon the substituents present in compounds of the formula I, the compounds may form esters, amides, and/or salts which are within the scope of the present invention. Salts and solvates of compounds of formula I which are suitable for use in medicine are those wherein a counterion or an associated solvent is pharmaceutically acceptable. However, salts and solvates having non-pharmaceutically acceptable counterions or associated solvents are within the scope of the present invention, for example, for use as intermediates in the preparation of the compounds of formula I and their pharmaceutically acceptable salts, solvates, and physiologically functional derivatives. By the term "physiologically functional derivative" is meant a chemical derivative of a compound of formula I having the same physiological function as the free compound of formula I, for example, by being convertible in the body thereto. Esters and amides are examples of physiologically functional derivatives.

A compound which, upon administration to the recipient, is capable of being converted into a compound of formula I as described above, or an active metabolite or residue thereof, is known as a "prodrug". A prodrug may, for example, be converted within the body, e. g. by hydrolysis in the blood, into its active form that has medical effects. Pharmaceutical acceptable prodrugs are described in T. Higuchi and V. Stella, Prodrugs as Novel Delivery Systems, Vol. 14 of the A. C. S. Symposium Series (1976); "Design of Prodrugs" ed. H. Bundgaard, Elsevier, 1985; and in Edward B. Roche, ed., Bioreversible Carriers in Drug Design, American Pharmaceutical Association and Pergamon Press, 1987, which are incorporated herein by reference.

Suitable salts according to the invention include those formed with organic or inorganic acids or bases. In particular, suitable salts formed with acids according to the invention include those formed with mineral acids, strong organic carboxylic acids, such as alkanecarboxylic acids of 1 to 4 carbon atoms which are unsubstituted or substituted, for example, by halogen, such as saturated or unsaturated dicarboxylic acids, such as hydroxycarboxylic acids, such as amino acids, or with organic sulfonic acids, such as (C₁-C₄)alkyl- or aryl-sulfonic acids which are unsubstituted or substituted, for example by halogen. Pharmaceutically acceptable acid addition salts include those formed from hydrochloric, hydrobromic, sulphuric, nitric, citric, tartaric, acetic, phosphoric, lactic, pyruvic, acetic, trifluoroacetic, succinic, perchloric, fumaric, maleic, glycolic, lactic, salicylic, oxaloacetic, methanesulfonic, ethanesulfonic, p-toluenesulfonic, formic, benzoic, malonic, naphthalene-2-sulfonic, benzenesulfonic, isethionic, ascorbic, malic, phthalic, aspartic, and glutamic acids, lysine, and arginine. Other acids such as oxalic, while not in themselves pharmaceutically acceptable, may be useful as intermediates in obtaining the compounds of the invention and their pharmaceutical acceptable acid addition salts.

Pharmaceutically acceptable base salts include ammonium salts, alkali metal salts, for example those of potassium and sodium, alkaline earth metal salts, for example those of calcium and magnesium, and salts with organic bases, for example dicyclohexylamine, *N*-methyl-D-glucamine, morpholine, thiomorpholine, piperidine, pyrrolidine, a mono-, di- or tri-lower alkylamine, for example ethyl-, tert-butyl-, diethyl-, diisopropyl-, triethyl-, tributyl- or dimethyl-propylamine, or a mono-, di- or trihydroxy lower alkylamine, for example mono-, di- or triethanolamine. Corresponding internal salts may furthermore be formed.

Those skilled in the art of organic chemistry will appreciate that many organic compounds can form complexes with solvents in which they are reacted or from which they are precipitated or crystallized. These complexes are known as "solvates". For example, a complex with water is known as a "hydrate".

The following definitions apply to the terms as used throughout this specification, unless otherwise limited in specific instances.

As used herein, the term "alkyl" means both straight and branched chain saturated hydrocarbon groups. Examples of alkyl groups include methyl, ethyl, n-propyl, iso-propyl, n-butyl, t-butyl, iso-butyl, and sec-butyl groups. Among unbranched alkyl groups, there are preferred methyl, ethyl, n-propyl, and n-butyl groups. Among branched alkyl groups, there may be mentioned iso-propyl, t-butyl, iso-butyl, and sec-butyl groups.

As used herein, the term "alkoxy" means the group O-alkyl, where "alkyl" is used as described above. Examples of alkoxy groups include, but are not limited to, methoxy and ethoxy groups. Other examples include propoxy and butoxy, such as isopropoxy and tert-butoxy.

As used herein, the term "halogen" means fluorine, chlorine, bromine or iodine. Fluorine, chlorine, and bromine are particularly preferred.

As used herein, the term "heterocyclyl" means a cyclic group of carbon atoms wherein from one to three of the carbon atoms is/are replaced by one or more heteroatoms independently selected from nitrogen, oxygen, and sulfur.

Examples of heterocyclyl groups include, but are not limited to, tetrahydrofuranyl, pyrrolidinyl, piperidinyl, piperazinyl, morpholinyl, and dioxanyl.

The compounds of the invention may be used in the prophylaxis and treatment as such, or preferably in a form of a pharmaceutical composition. While it is possible for the active ingredient to be administered alone, it is preferable for it to be present in a pharmaceutical formulation or composition. Accordingly, the invention provides a pharmaceutical formulation comprising a compound according to the invention, and a pharmaceutically acceptable diluent, excipient or carrier (collectively referred to herein as "carrier" materials). Pharmaceutical compositions of the invention may take the form of a pharmaceutical formulation as described below. Thus, the present invention relates to a pharmaceutical composition containing at least one compound of Formula I together with conventional excipients.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate, calcium sulfate, sorbitol, glucose, and/or lactose and/or other excipients, binders, extenders, disintegrants, diluents, and lubricants such as those known in the art. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes, and the like. Disintegrators include without limitation starch, methylcellulose, agar, bentonite, xanthan gum, and the like. The compounds of formula I can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose, and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents, and stabilizers may also be added for ease of fabrication and use. Lubricants used in these dosage forms include sodium oleate, sodium stearate, magnesium stearate, sodium benzoate, sodium acetate, sodium chloride, and the like. For oral administration in liquid form, the oral drug components can be combined with any oral, non-toxic, pharmaceutically acceptable inert carrier such as ethanol, glycerol, water, and the like.

The pharmaceutical formulations according to the invention include those suitable for oral, parenteral [including subcutaneous, intradermal, intramuscular, intravenous (bolus or infusion), and intraarticular], inhalation (including fine particle dusts or mists which may be generated by means of various types of metered dose pressurized aerosols), nebulizers or insufflators, rectal, intraperitoneal, and topical (including dermal, buccal, sublingual, and intraocular) administration, although the most suitable route may depend upon, for example, the condition and disorder of the recipient.

Formulations of the present invention suitable for oral administration may be presented as discrete units such as capsules, cachets, pills or tablets each containing a predetermined amount of the active ingredient; as a powder or granules; as a solution or a suspension in an aqueous liquid or a non-aqueous liquid, for example as elixirs, tinctures, suspensions or syrups; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally. Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

The compounds of the present invention can also be administered in the form of liposome delivery systems, such as small unilamellar vesicles, large unilamellar vesicles, and multilamellar vesicles. Liposomes can be formed from a variety of phospholipids, 1,2-dipalmitoylphosphatidylcholine, phosphatidyl ethanolamine (cephaline), phosphatidylserine, phosphatidylinositol, diphosphatidylglycerol 1 (cardiolipin) or phosphatidylcholine (lecithin).

Formulations for parenteral administration include aqueous and non-aqueous sterile injection solutions which may contain anti-oxidants, buffers, bacteriostats, and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules, and tablets of the kind previously described. Exemplary compositions for parenteral administration include injectable solutions or suspensions which can contain, for example, suitable non-toxic, parenterally acceptable diluents or solvents, such as polyethylene glycol, ethanol, 1,3-butanediol, water, Ringer's solution, an isotonic sodium chloride solution, or other suitable dispersing or wetting and suspending agents, including synthetic mono- or diglycerides, and fatty acids, including oleic acid and Cremaphor.

Exemplary compositions for nasal, aerosol or inhalation administration include solutions in saline, which can contain, for example, benzyl alcohol or other suitable preservatives, absorption promoters to enhance bioavailability, and/or other solubilizing or dispersing agents such as those known in the art.

Formulations for rectal administration may be presented as a suppository with the usual carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

The amount of active ingredient which is required to achieve a therapeutic effect will, of course, vary with the particular compound, the route of administration, the subject under treatment, including the type, species, age, weight, sex, and medical condition of the subject and the renal and hepatic function of the subject, and the particular disorder or disease being treated, as well as its severity. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 10 mg/kg/day, and most preferably 0.1 to 5.0 mg/kg/day, for adult humans. For oral administration, the compositions are preferably provided in the form of tablets or other forms of presentation provided in discrete units containing 0.01, 0.05, 0.1, 0.5, 1.0, 2.5, 5.0, 10.0, 15.0, 25.0, 50.0, 100, and 500 milligrams of the active ingredient for the symptomatic adjustment of the dosage to the patient to be treated. A medicament typically contains from about 0.01 mg to about 500 mg of the active ingredient, preferably from about 1 mg to about 100 mg of active ingredient. Intravenously, the most preferred doses will range from about 0.1 to about 10 mg/kg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

The invention also provides the use of a compound of formula I, for the manufacture of a medicament for the treatment or prophylaxis of cancer.

The compounds and the pharmaceutical compositions of the invention may be used in the prophylaxis and treatment of diseases such as cancer, diseases caused by parasites, allergic diseases, Crohns disease, rheumatic diseases, tuberculosis, diabetes, Alzheimer's disease, inflammatory diseases, multiple sclerosis (MS), amyotrophic lateral sclerosis (ALS), Parkinson's disease and diseases caused by bacteria, viruses, and fungus.

The compounds of the invention find particular application in the treatment or prophylaxis of various proliferative disorders and cancer types, including but not limited to, cancer of the bone, breast, respiratory tract, brain, reproductive organs, digestive tract, urinary tract, eye, liver, skin, head, neck, thyroid, parathyroid, and metastatic forms thereof. Proliferative disorders of the breast include, but are not limited to, invasive ductal carcinoma, invasive lobular carcinoma, ductal carcinoma, lobular carcinoma in situ, and metastatic breast cancer. Proliferative disorders of the skin include, but are not limited to, basal cell carcinoma, squamous cell carcinoma, malignant melanoma, and Kaposi's sarcoma. Proliferative disorders of the respiratory tract include, but are not limited to, small cell and non-small cell lung carcinoma, bronchial adenoma, pleuropulmonary blastoma, and malignant mesothelioma. Proliferative disorders of the brain include, but are not limited to, brain stem and hypothalamic glioma, cerebellar and cerebral astrocytoma, medulloblastoma, ependymal tumors, oligodendroglial tumors, meningiomas and neuroectodermal, and pineal tumors. Proliferative disorders of the male reproductive organs include, but are not limited to, prostate, testicular, and penis cancer. Proliferative disorders of the female reproductive organs include, but are not limited to, uterine, cervical, ovarian, vaginal, vulval cancer, uterine sarcoma, and ovarian germ cell tumor. Proliferative disorders of the digestive tract include, but are not limited to, anal, colon, colorectal, esophageal, gall bladder, stomach, pancreatic, rectal, small intestine, and salivary gland cancer. Proliferative disorders of the liver include, but are not limited to, hepatocellular carcinoma, cholangiocarcinoma, and primary liver cancer. Proliferative disorders of the eye include, but are not limited to, intraocular melanoma, retinoblastoma, and rhabdomyosarcoma. Proliferative disorders of the head include, but are not limited to, laryngeal, hypopharyngeal, nasopharyngeal, oropharyngeal, lip, oral, and metastatic paranasal sinus cancer. Proliferative disorders of the lymphomas include, but are not limited to, T cell and B cell lymphomas, non-Hodgkins lymphoma, cutaneous T cell lymphoma, Hodgkins disease, and lymphoma of the central nervous system. Leukemia includes, but is not limited to, acute myeloid leukemia, chronic myelogenous leukemia, and hairy cell leukemia. Proliferative disorders of the thyroid include, but are not limited to, thyroid cancer, thymoma, and malignant thymoma. Proliferative disorders of the urinary tract include, but are not limited to, kidney cancer and bladder cancer. Sarcomas include, but are not limited to, sarcoma of the soft tissue, osteosarcoma, malignant fibrous histiocytoma, lymphosarcoma, and rhabdomyosarcoma.

Whilst a compound of the invention may be used alone, it is also possible for the compounds to be used in combination with each other, in combination with radiation therapy, or in combination with other anticancer agents. Various classes of anticancer and antineoplastic compounds include, but are not limited to, alkylating agents, antimetabolites, anticancer camptothecin derivatives, plant-derived anticancer agents, antibiotics, enzymes, platinum coordination complexes, tubulin inhibitors, tyrosine kinase inhibitors, hormones and hormone antagonists, monoclonal antibodies, interferons, biological response modifiers and other anticancer agents. Examples of alkylating agents include, but are not limited to, mechlorethamine, cyclophosphamide, ifosfamide, melphalan, chlorambucil, busulfan, mitobronitol, ranimustin, nimustin, temozolomide, and carmustine; examples of antimetabolites include, but are not limited to, methotrexate, fluorouracil, cytarabine, gemcitabine, fludarabine, mercaptopurine, thioguanine, and azathioprine; examples of camptothecin derivatives include, but are not limited to, irinotecan, topotecan, and camptothecin; examples of plant-derived agents include, but are not limited to, vinblastine, vincristine, docetaxel, paclitaxel, and colchicines; examples of antibiotics include, but are not limited to, actinomycin D, daunorubicin, and bleomycin. One example of enzyme effective as antineoplastic agent includes L-asparaginase. Examples of coordination compounds include, but are not limited to, cisplatin and carboplatin; examples of tubulin inhibitors include, but are not limited to, the above mentioned plant-derived agents; examples of tyrosine kinase inhibitors include, but are not limited to, gefitinib, imatinib, sunitinib, nilotinib, dasatinib, erlotinib, and pazopanib; examples of hormones and hormone related compounds include, but are not limited to, prednisone, dexamethasone, formestane, aminoglutethimide, anastrozole, hydroxyprogesterone caproate, medroxyprogesterone, and tamoxifen; examples of interferons include, but are not limited to, interferon α,interferon α-2a, interferon α-2b, interferon β, interferon γ-la, and interferon γ-nl; examples of biological response modifiers include, but are not limited to, krestin, lentinan, sizofiran, picibanil, and ubenimex. Examples of other anticancer agents include, but are not limited to, mitoxantrone, procarbazine, dacarbazine, hydroxycarbamide, pentostatin, tretinoin, leuprorelin, flutamide, and aldesleukin.

Numerous synthetic routes to the compounds of the present invention can be devised by any person skilled in the art and the possible synthetic routes described below do not limit the invention.

### Procedures for synthesizing compounds of general formula I

### Method A

The appropriate amine (II or V) was dissolved in isopropanol (0.2 g/mL). 1.1 Eq of pyrimidine (III) and 1.2 eq of *N,N* diisopropylethylamine (DIPEA) were added and the mixture was stirred at temperatures between 25 °C and 80 °C for 1 h. The reaction mixture was dissolved in EtOAc/MeOH 9:1 and washed with saturated aqueous NaHCO₃, water, and brine. The solvent was removed *in vacuo* and the residue was purified by column chromatography on silica gel with heptane/EtOAc, EtOAc and/or EtOAc/MeOH as eluent to give the intermediate (IV' or IV").

The intermediate (IV' or IV") was dissolved in ethylene glycol (0.2 g/mL) and 1.3 eq of amine (II or V) and 1.3 eq of *N*,*N* diisopropylethylamine (DIPEA) were added. The mixture was then stirred at temperatures between 100 and 150°C for 1-3 h. The reaction mixture was dissolved in EtOAc/MeOH 9:1 and washed with saturated aqueous NaHCO₃, water, and brine. The solvent was removed *in vacuo* and the residue was purified by column chromatography on silica gel with heptane/EtOAc, EtOAc and/or EtOAc/MeOH/TEA as eluent to give the compound of formula 1. This procedure is exemplified in Scheme 1.

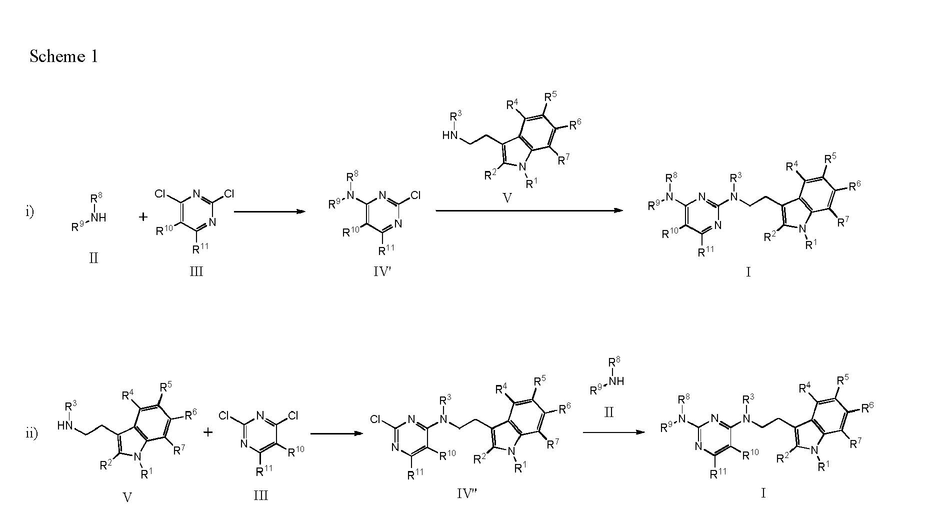

Compounds of Examples 1-29 were synthesized by Method A (as set out in Scheme 1). R¹-R¹¹ are as defined in formula I.

### Method B

The appropriate amine (V) was dissolved in isopropanol/N-methyl-2-pyrrolidone (0.2 g/mL). 1.0 eq of pyrimidine (III), 1.0 eq of *N*,*N*-diisopropylethylamine (DIPEA), and 1.2 eq of NaI were added and the mixture was stirred at 150 °C for 12 h. The reaction mixture was dissolved in EtOAc/MeOH 9:1 and washed with saturated aqueous NaHCO₃, water, and brine. The solvent was removed *in vacuo* and the residue was purified by column chromatography on silica gel with EtOAc/MeOH/TEA as eluent to give the intermediate (IV"').

Intermediate (IV"') was added to a solution of 0.10 eq Pd(OAc)₂, 0.15 eq 2,2'-bis(diphenylphosphino)-1,1'-binaphthyl (BINAP), and 1.5 eq CsCO₃ in N-methyl-2-pyrrolidone (NMP) (0.2 g/mL) under an atmosphere of argon. The mixture was then stirred at 150 °C for 1 h. The reaction mixture was dissolved in EtOAc/MeOH 9:1 and washed with saturated aqueous NaHCO₃, water, and brine. The solvent was removed *in vacuo* and the residue was purified by column chromatography on silica gel with EtOAc/MeOH/TEA as eluent to give the compound of formula 1. This procedure is exemplified in Scheme 2.

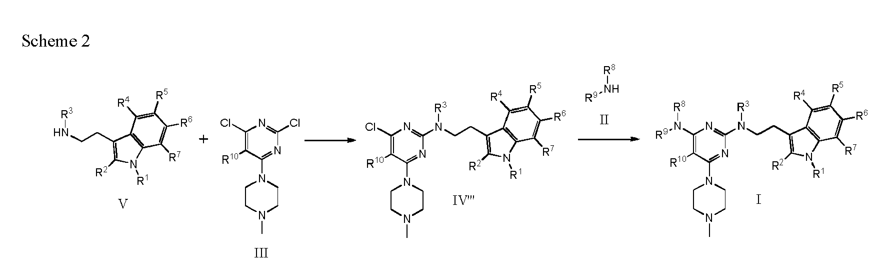

Example 30 was synthesized by Method B (as set out in Scheme 2). R¹-R¹⁰ are as defined in formula 1.

### Example 1

### N²-[2-(1H-indol-3-yl)ethl]-N⁴-(1H-indol-5-yl)pyrimidine-2,4-diamine

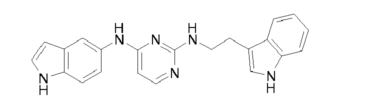

¹H NMR (500 MHz, DMSO-*d*₆, 75°C) δ 10.77 (s, 1H), 10.61 (s, 1H), 8.58 (s, 1H), 7.81 (s, 1H), 7.79 (d, 1H), 7.57 (d, 1H), 7.34 (d, 1H), 7.30 (d, 1H), 7.25 (m, 1H), 7.20 (d, 1H), 7.14 (s, 1H), 7.06 (t, 1H), 6.96 (t, 1H), 6.30 (s, 1H), 6.24 (m, 1H), 5.93 (d, 1H), 3.60 (q, 2H), 3.00 (t, 2H).

MS (ESI⁺) *m*/*z* 369.3 [M + H]⁺.

### Example 2

### N⁴-(1H-indol-5-yl)-N²-[2-(5-methoxy-1H-indol-3-yl)ethyl]pyrimidine-2,4-diamine

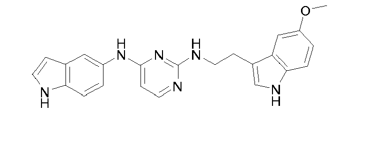

¹H NMR (500 MHz, DMSO-*d*₆, 75°C) δ 10.77 (s, 1H), 10.45 (s, 1H), 8.57 (s, 1H), 7.82 (s, 1H), 7.79 (d, 1H), 7.29 (d, 1H), 7.25-7.18 (m, 3H), 7.10 (s, 1H), 7.08 (s, 1H), 6.72 (dd, 1H), 6.30 (s, 1H), 6.22 (m, 1H), 5.94 (d, 1H), 3.75 (s, 3H), 3.58 (q, 2H), 2.95 (t, 2H).

MS (ESI⁺) *m*/*z* 399.3 [M + H]⁺.

### Example 3

### N²-[2-(1H-indol-3-yl)ethl]-N⁴-(2-methyl-1H-indol-5-yl)pyrimidine-2,4-diamine

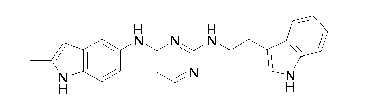

¹H NMR (500 MHz, DMSO-*d*₆) δ 10.37 (s, 1H), 10.34 (s, 1H), 8.18 (br. s, 1H), 7.76 (d, 1H), 7.58 (d, 1H), 7.56 (br. s, 1H), 7.36 (d, 1H), 7.20 (d, 1H), 7.09-7.06 (m, 3H), 7.00 (t, 1H), 6.02 (s, 1H), 5.94 (d, 1H), 5.54 (br. s, 1H); 3.67 (q, 2H), 3.04 (t, 2H), 2.41 (s, 3H). MS (ESI⁺) m/z 383.2 [M + H]⁺.

### Example 4

### N²-[2-(5-methoxy-1H-indol-3-yl)ethyl]-N⁴-(2-methyl-1H-indol-5-yl)pyrimidine-2,4-diamine

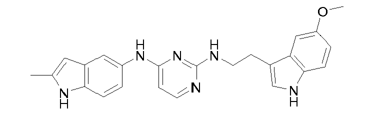

¹H NMR (500 MHz, DMSO-*d*₆, 75°C) *δ* 10.45 (s, 1H), 8.50 (s, 1H), 7.77 (d, 1H), 7.65 (s, 1H), 7.23 (d, 1H), 7.17 (d, 1H), 7.11-7.07 (m, 3H), 6.72 (dd, 1H), 6.20 (m, 1H), 5.97 (s, 1H), 5.91 (d, 1H), 3.75 (s, 3H), 3.58 (q, 2H), 2.95 (t, 2H), 2.36 (s, 3H). MS (ESI⁺) *m*/*z* 413.4 [M + H]⁺.

### Example 5

### N²-[2-(5-ethoxy-1H-indol-3-yl)ethl]-N⁴-(2-methyl-1H-indol-5-yl)pyrimidine-2,4-diamine

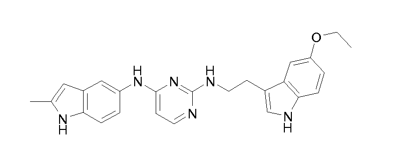

¹H NMR (500 MHz, DMSO-*d*₆, 75°C) δ 10.56 (s, 1H), 10.43 (s, 1H), 8.50 (s, 1H), 7.77 (d, 1H), 7.65 (s, 1H), 7.22 (d, 1H), 7.15 (d, 1H), 7.09-7.06 (m, 3H), 6.71 (dd, 1H), 6.19 (m, 1H), 5.97 (s, 1H), 5.91 (d, 1H), 4.01 (q, 2H), 3.57 (q, 2H), 2.93 (t, 2H), 2.36 (s,3H), 1.31(t,3H).

MS (ESI⁺) *m*/*z* 427.3 [M + H]⁺.

### Example 6

### N⁴-(2-methyl-1H-indol-5-yl)-N²-{2-[5-(2-morpholinoethoxy)-1H-indol-3-yl]ethyl}pirimidine-2,4-diamine

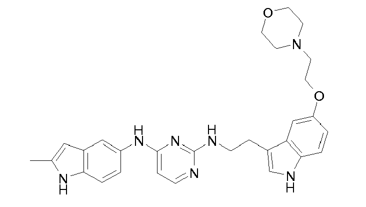

¹H NMR (500 MHz, DMSO-d₆, 75°C) δ 10.56 (s, 1H), 10.45 (s, 1H), 8.50 (s, 1H), 7.77 (d, 1H), 7.66 (s, 1H), 7.22 (d, 1H), 7.15 (d, 1H), 7.10-7.07 (m, 3H), 6.72 (dd, 1H), 6.19 (m, 1H), 5.96 (s, 1H), 5.91 (d, 1H), 4.07 (t, 2H), 3.57 (m, 6H), 2.94 (t, 2H), 2.68 (t, 2H), 2.47 (m, 4H), 2.35 (s, 3H).

MS (ESI⁺) *m*/*z* 512.4 [M + H]⁺.

### Example 7

### N⁴-(2-methyl-1H-indol-5-yl)-N²-{2-[5-(trifluoromethoxy)-1H-indol-3-yl]ethyl}pirimidine-2,4-diamine

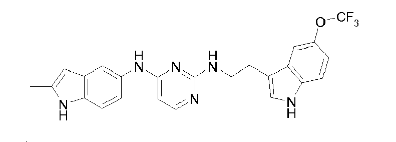

¹H NMR (500 MHz, DMSO-*d*₆, 75°C) δ 10.93 (s, 1H), 10.56 (s, 1H), 8.50 (s, 1H), 7.77 (d, 1H), 7.64 (s, 1H), 7.53 (s, 1H), 7.42 (d, 1H), 7.28 (s, 1H), 7.16 (d, 1H), 7.08 (dd, 1H), 7.02 (d, 1H), 6.26 (br s, 1H), 5.97 (s, 1H), 5.92 (d, 1H), 3.57 (q, 2H), 2.97 (t, 2H), 2.36 (s, 3H).

MS (ESI⁺) *m*/*z* 467.2 [M + H]⁺.

### Example 8

### 3-{2-[4-(2-methyl-1H-indol-5-ylamino)pyrimidin-2-ylamino]ethyl}-1H-indol-5-ol

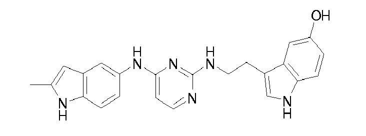

¹H NMR (500 MHz, CD₃OD) *δ* 7.66 (d, 1H), 7.52 (br s, 1H), 7.20-7.14 (m, 2H), 7.03 (br s, 1H), 7.01 (s, 1H), 6.97 (s, 1H), 6.66 (dd, 1H), 6.01 (s, 1H), 5.90 (d, 1H), 3.63 (t, 2H), 2.96 (t, 2H), 2.38 (s, 3H).

MS (ESI⁺) *m*/*z* 399.3 [M + H]⁺.

### Example 9

### N²-[2-(5-methyl-1H-indol-3-yl)ethyl]-N⁴-(2-methyl-1H-indol-5-yl)pyrimidine-2,4-diamine

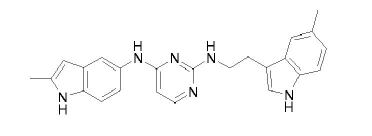

¹H NMR (500 MHz, DMSO-*d*₆, 75°C) *δ* 10.56 (s, 1H), 10.46 (s, 1H), 8.50 (s, 1H), 7.77 (d, 1H), 7.65 (s, 1H), 7.34 (s, 1H), 7.23 (d, 1H), 7.15 (d, 1H), 7.11-7.08 (m, 2H), 6.89 (d, 1H), 6.18 (m, 1H), 5.97 (s, 1H), 5.91 (d, 1H), 3.58 (q, 2H), 2.95 (t, 2H), 2.37 (s, 3H), 2.36 (s, 3H).

MS (ESI⁺) *m*/*z* 397.3 [M + H]⁺.

### Example 10

### N²-[2-(5-fluoro-1H-indol-3-yl)ethyl]-N⁴-(2-methyl-1H-indol-5-yl)pyrimidine-2,4-diamine

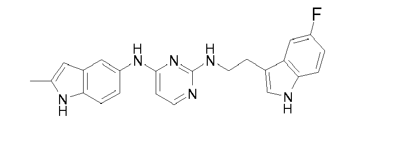

¹H NMR (500 MHz, DMSO-*d*₆,) *δ* 10.90 (s, 1H), 10.73 (s, 1H), 8.75 (br s, 1H), 7.74 (m, 2H), 7.32 (m, 2H), 7.24 (s, 1H), 7.13 (d, 1H), 7.08 (d, 1H), 6.90 (t, 1H), 6.53 (br s, 1H), 5.90 (d, 2H), 3.53 (m, 2H), 2.92 (m, 1H), 2.33 (s, 3H).

MS (ESI⁺) *m*/*z* 401.3 [M + H]⁺.

### Example 11

### N²-[2-(6-methoxy-1H-indol-3-yl)ethyl]-N⁴-(2-methyl-1H-indol-5-yl)pyrimidine-2,4-diamine

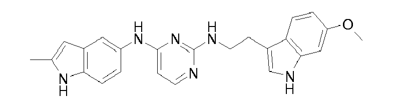

¹H NMR (500 MHz, DMSO-*d*₆, 75°C) *δ* 10.56 (s, 1H), 10.40 (s, 1H), 8.50 (s, 1H), 7.77 (d, 1H), 7.64 (s, 1H), 7.42 (d, 1H), 7.16 (d, 1H), 7.09 (d, 1H), 6.99 (s, 1H), 6.86 (s, 1H), 6.63 (d, 1H), 6.17 (t, 1H), 5.98 (s, 1H), 5.90 (d, 1H), 3.77 (s, 3H), 3.57 (q, 2H), 2.92 (t, 2H), 2.36 (s, 3H).

MS (ESI⁺) *m*/*z* 413.3 [M + H]⁺.

### Example 12

### N²-[2-(7-methoxy-1H-indol-3-yl)ethyl]-N⁴-(2-methyl-1H-indol-5-yl)pyrimidine-2,4-diamine

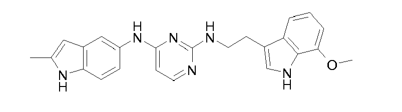

¹H NMR (500 MHz, DMSO-*d*₆, 75°C) *δ* 10.65 (s, 1H), 10.57 (s, 1H), 8.50 (s, 1H), 7.76 (d, 1H), 7.64 (s, 1H), 7.18-7.15 (m, 2H), 7.10 (m, 1H), 7.06 (m, 1H), 6.89 (t, 1H), 6.64 (d, 1H), 6.19 (m, 1H), 5.97 (s, 1H), 5.90 (d, 1H), 3.91 (s, 3H), 3.57 (q, 2H), 2.95 (t, 2H), 2.36 (s, 3H).

MS (ESI⁺) *m*/*z* 413.3 [M + H]⁺.

### Example 13

### N⁴-(1,2-dimethyl-1H-indol-5-yl)-N²-[2-(5-methoxy-1H-indol-3-yl)ethyl)pyrimidine-2,4-diamine

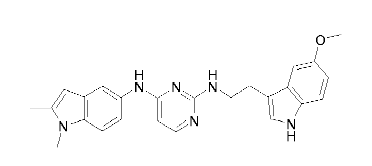

¹H NMR (500 MHz, DMSO-*d*₆, 75°C) *δ* 10.45 (s, 1H), 8.56 (s, 1H), 7.78 (d, 1H), 7.70 (s, 1H), 7.25-7.20 (m, 3H), 7.10 (d, 1H), 7.07 (d, 1H), 6.73 (dd, 1H), 6.23 (br s, 1H), 6.05 (s, 1H), 5.92 (d, 1H), 3.74 (s, 3H), 3.62 (s, 3H), 3.58 (q, 2H), 2.95 (t, 2H), 2.37 (s, 3H).

MS (ESI⁺) *m*/*z* 427.3 [M + H]⁺.

### Example 14

### N⁴-(2,3-dimethyl-1H-indol-5-yl)-N²-[2-(5-methoxy-1H-indol-3-yl)ethyl]pyrimidine-2,4-diamine

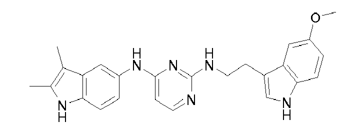

¹H NMR (500 MHz, DMSO-*d*₆, 75°C) *δ* 10.44 (s, 1H), 10.31 (s, 1H), 8.52 (s, 1H), 7.77 (d, 1H), 7.63 (s, 1H), 7.23 (d, 1H), 7.13-7.05 (m, 4H), 6.72 (dd, 1H), 6.15 (m, 1H), 5.91 (d, 1H), 3.74 (s, 3H), 3.60 (q, 2H), 2.95 (t, 2H), 2.29 (s, 3H), 2.07 (s, 3H). MS (ESI⁺) *m*/*z* 427.3 [M + H]⁺.

### Example 15

### (5-{2-[2-(5-methoxy-1H-indol-3-yl)ethylamino]pyrimidin-4-ylamino}-1H-indol-2-yl)methanol

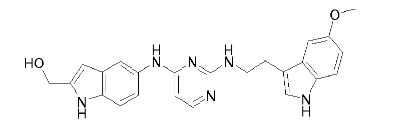

¹H NMR (500 MHz, DMSO-*d*₆, 75°C) *δ* 10.65 (s, 1H), 10.44 (s, 1H), 8.53 (s, 1H), 7.78 (d, 1H), 7.72 (s, 1H), 7.23 (d, 2H), 7.15 (dd, 1H), 7.10 (d, 1H), 7.08 (d, 1H), 6.72 (dd, 1H), 6.20 (t, 1H), 6.15 (s, 1H), 5.92 (d, 1H), 4.95 (t, 1H), 4.59 (d, 2H), 3.75 (s, 3H), 3.58 (q, 2H), 2.95 (t, 2H).

MS (ESI⁺) *m*/*z* 429.4 [M + H]⁺.

### Example 16

### melhyl 5-{2-[2-(5-methoxy-1H-indol-3-yl)ethylamino]pyrimidin-4-ylamino}-1H-indo le-2-carboxylate

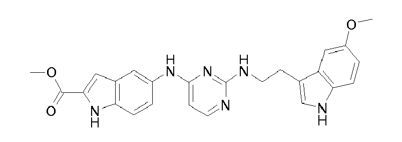

¹H NMR (500 MHz, DMSO-*d*₆, 75°C) *δ* 11.56 (s, 1H), 10.44 (s, 1H), 8.74 (s, 1H), 8.03 (s, 1H), 7.82 (d, 1H), 7.41-7.35 (m, 2H), 7.23 (d, 1H), 7.10 (d, 1H), 7.07 (d, 1H), 6.96 (s, 1H), 6.72 (dd, 1H), 6.30 (t, 1H), 5.97 (d, 1H), 3.87 (s, 3H), 3.73 (s, 3H), 3.60 (q, 2H), 2.96 (t, 2H).

MS (ESI⁺) *m*/*z* 457.3 [M + H]⁺.

### Example 17

### 2-hydroxyethyl 5-{2-[2-(5-methoxy-1H-indol-3-yl)ethylamino]pyrimidin-4-ylamino}-1H-indole-2-carboxylate

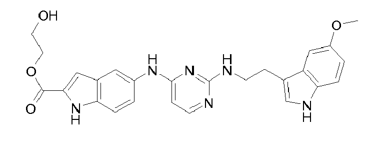

¹H NMR (500 MHz, DMSO-*d*₆, 75°C) *δ* 11.51 (s, 1H), 10.44 (s, 1H), 8.75 (s, 1H), 8.04 (s, 1H), 7.82 (d, 1H), 7.42-7.36 (m, 2H), 7.23 (d, 1H), 7.10 (d, 1H), 7.07 (d, 1H), 7.01 (s, 1H), 6.72 (dd, 1H), 6.31 (m, 1H), 5.97 (d, 1H), 4.71 (m, 1H), 4.32 (t, 2H), 3.75 (m, 2H), 3.74 (s, 3H), 3.60 (q, 2H), 2.96 (t, 2H).

MS (ESI⁺) *m*/*z* 487.2 [M + H]⁺.

### Example 18

### N⁴-(1H-benzo[d]imidazol-5-yl)-N²-[2-(5-methoxy-1H-indol-3-yl)ethyl]pyrimidine-2,4-diamine

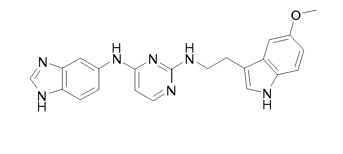

¹H NMR (500 MHz, DMSO-*d₆*, 75°C) *δ* 12.07 (br s, 1H), 10.45 (s, 1H), 8.84 (br s, 1H), 8.05 (s, 1H), 8.00-7.88 (m, 1H), 7.83 (d, 1H), 7.47 (m, 1H), 7.38 (d, 1H), 7.22 (d, 1H), 7.12 (br s, 1H), 7.07 (s, 1H), 6.71 (d, 1H), 6.29 (br s, 1H), 6.00 (d, 1H), 3.74 (s, 3H), 3.59 (q, 2H), 2.95 (t, 3H).

MS (ESI⁺) m/z 400.3 [M + H]⁺.

### Example 19

### N²-[2-(5-methoxy-1H-indol-3-yl)ethyl]-N⁴-(2-methyl-1H-benzo[d]imidazol-5-yl)pyrimidine-2,4-diamine

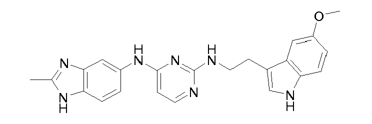

¹H NMR (500 MHz, DMSO-d₆, 75°C) *δ* 11.76 (br s, 1H), 10.45 (s, 1H), 8.75 (br s, 1H), 7.82 (d, 1H), 7.78 (br s, 1H), 7.34-7.28 (m, 2H), 7.23 (d, 1H), 7.13 (br s, 1H), 7.07 (d, 1H), 6.72 (dd, 1H), 6.26 (br s, 1H), 5.97 (d, 1H), 3.74 (s, 3H), 3.59 (q, 2H), 2.95 (t, 2H), 2.46 (s, 3H).

MS (ESI⁺) m/z 414.3 [M + H]⁺.

### Example 20

### N⁴-(1H-indol-6-yl)-N²-[2-(5-methoxy-1H-indol-3-yl)ethyl[pyrimidine-2,4-diamine

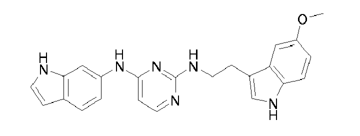

¹H NMR (500 MHz, DMSO-*d₆*, 75°C) *δ* 10.66 (s, 1H), 10.45 (s, 1H), 8.71 (s, 1H), 7.82 (d, 1H), 7.71 (s, 1H), 7.41 (d, 1H), 7.23 (d, 1H), 7.21-7.19 (m, 2H), 7.10 (d, 1H), 7.08 (d, 1H), 6.72 (dd, 1H), 6.35 (s, 1H), 6.18 (m, 1H), 6.00 (d, 1H), 3.75 (s, 3H), 3.61 (q, 2H), 2.96 (t, 2H).

MS (ESI⁺) m/z 399.2 [M + H]⁺.

### Example 21

### N⁴-(1H-indol-4-yl)-N²-[2-(5-methoxy-1H-indol-3-yl)ethyl]pyrimidine-2,4-diamine

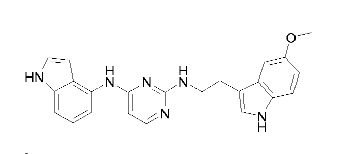

¹H NMR (500 MHz, DMSO-*d*₆, 75°C) *δ* 10.89 (s, 1H), 10.45 (s, 1H), 8.51 (s, 1H), 7.85 (d, 1H), 7.66 (d, 1H), 7.24-7.22 (m, 2H), 7.11-7.07 (m, 3H), 6.98 (t, 1H), 6.72 (dd, 1H), 6.64 (m, 1H), 6.30 (m, 1H), 6.13 (d, 1H), 3.75 (s, 3H), 3.58 (q, 2H), 2.94 (t, 2H).

MS (ESI⁺) *m*/*z* 399.3 [M + H]⁺.

### Example 22

### N⁴-[2-(1H-indol-3-yl)ethyl-N²-(2-methyl-1H-indol-5-yl)pyrimidine-2,4-diamine

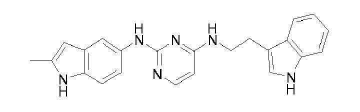

¹H NMR (300 MHz, DMSO-*d*₆) *δ* 10.81 (br. s, 1H), 10.57 (br. s, 1H), 8.47 (s, 1H), 7.85 (s, 1H), 7.73 (br. s, 1H), 7.52 (d, 1H), 7.33 (d, 1H), 7.22 (d, 1H), 7.16 (s, 1H), 7.11 (br. S, 1H), 7.06 (t, 2H), 6.95 (t, 1H), 5.84 (d, 2H), 3.61 (br. s, 2H), 2.96 (t, 1H), 2.30 (s, 3H).

MS (ESI⁺) *m*/*z* 383.3 [M + H]⁺.

### Example 23

### N⁴-(2-(5-methoxy-1H-indol-3-yl)ethyl)-N²-(2-methyl-1H-indol-5-yl)pyrimidine-2,4-diamine

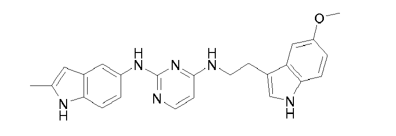

¹H NMR (500 MHz, DMSO-*d*₆, 75°C) *δ* 10.48 (s, 1H), 10.40 (s, 1H), 8.18 (s, 1H), 7.83 (d, 1H), 7.76 (d, 1H), 7.24 (d, 2H), 7.12 (d, 1H), 7.07 (d, 1H), 7.02 (d, 1H), 6.80 (br s, 1H), 6.73 (dd, 1H), 5.90 (s, 1H), 5.87 (d, 1H), 3.74 (s, 3H), 3.61 (q, 2H), 2.97 (t, 2H), 2.34 (s, 3H).

MS (ESI⁺) *m*/*z* 413.3 [M + H]⁺.

### Example 24

### N⁴-(2-(1H-indol-3-yl)ethyl)-N²-(1H-indol-6-yl)pyrimidine-2,4-diamine

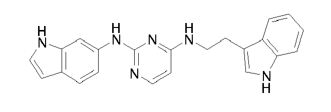

¹H NMR (500 MHz, DMSO-*d*₆) *δ* 10.82 (s, 1H), 10.78 (s, 1H), 8.72 (s, 1H), 8.06 (s, 1H), 7.78 (br s, 1H), 7.54 (d, 1H)7.35-7.30 (m, 2H), 7.26 (d, 1H), 7.20-7.10 (m, 3H), 7.06 (t, 1H), 6.95 (t, 1H), 6.29 (s, 1H), 5.90 (d, 1H), 3.65 (br s, 2H), 2.99 (t, 2H). MS (ESI⁺) *m*/*z* 369.2 [M + H]⁺.

### Example 25

### N⁴-(2-(1H-indol-3-yl)ethyl)-N²-(1H-indol-4-yl)pyrimidine-2,4-diamine

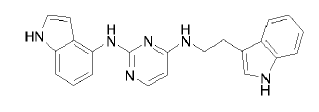

¹H NMR (500 MHz, CDCl₃) *δ* 8.21 (s, 1H), 8.06 (s, 1H), 8.02 (d, 1H), 7.95 (d, 1H), 7.63 (d, 1H), 7.38 (d, 1H), 7.24-7.07 (m, 6H), 7.03 (s, 1H), 6.58 (s, 1H), 5.82 (d, 1H), 4.85 (br s, 1H), 3.72 (br s, 2H), 3.10 (t, 2H).

MS (ESI⁺) *m*/*z* 369.2 [M + H]⁺.

### Example 26

### N²-[2-(1H-indol-3-yl)ethyl-6-methyl-N⁴(2-methyl-1H-indol-5-yl)pyrimidine-2,4-diamine

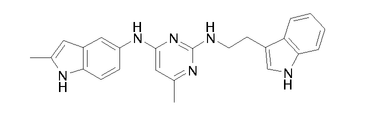

¹H NMR (500 MHz, DMSO-*d*₆,75°C) *δ* 10.61 (s, 1H), 10.55 (s, 1H), 8.35 (s, 1H), 7.59 (m, 2H), 7.35 (d, 1H), 7.18-7.13 (m, 2H), 7.09-7.06 (m, 2H), 6.96 (t, 1H), 6.12 (m, 1H), 5.97 (s, 1H), 5.78 (s, 1H), 3.59 (q, 2H), 2.97 (t, 2H), 2.36 (s, 3H), 2.08 (s, 3H). MS (ESI⁺) *m*/*z* 397.4 [M + H]⁺.

### Example 27

### N²-[2-(5-methoxy-1H-indol-3-yl)ethyl]-6-methyl-N⁴-(2-methyl-1H-indol-5-yl)pyrimidine-2,4-diamine

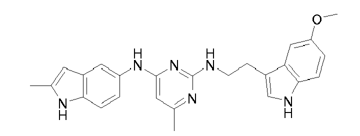

¹H NMR (500 MHz, DMSO-*d*₆, 75°C) *δ* 10.55 (s, 1H), 10.45 (s, 1H), 8.35 (s, 1H), 7.61 (s, 1H), 7.23 (d, 1H), 7.15 (d, 1H), 7.11 (d, 1H), 7.08-7.06 (m, 2H), 6.72 (dd, 1H), 6.10 (m, 1H), 5.97 (s, 1H), 5.78 (s, 1H), 3.75 (s, 3H), 3.58 (q, 2H), 2.94 (t, 2H), 2.35 (s, 3H), 2.08 (s, 3H).

MS (ESI⁺) *m*/*z* 427.3[M + H]⁺.

### Example 28

### 2-[2-(5-methoxy-1H-indol-3-yl)ethylaminol-6-(2-methyl-1H-indol-5-ylamino)pirimidine-4-carboxamide

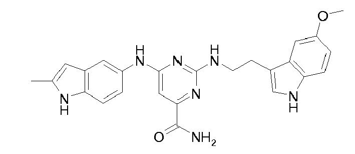

¹H NMR (500 MHz, DMSO-*d*₆,75°C) *δ* 10.60 (s, 1H), 10.46 (s, 1H), 8.88 (s, 1H), 7.68 (s, 1H), 7.56 (br s, 1H), 7.24 (m, 1H), 7.23 (d, 1H), 7.18 (d, 1H), 7.11 (m, 2H), 7.03 (s, 1H), 6.73 (d, 1H), 6.56 (s, 1H), 6.42 (m, 1H), 5.99 (br s, 1H), 3.74 (s, 3H), 3.34 (q, 2H), 2.97 (t, 2H), 2.36 (s, 3H).

MS (ESI⁺) *m*/*z* 456.3 [M + H]⁺.

### Example 29

### N²-[2-(5-methoxy-1H-indol-3-yl)ethyl]-5-methyl-N⁴-(2-methyl-1H-indol-5-yl)pyrimidme-2,4-diamine

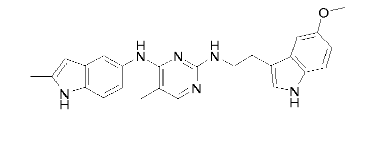

¹H NMR (500 MHz, DMSO-*d*₆, 75°C) *δ* 10.51 (s, 1H), 10.41 (s, 1H), 7.75 (s, 1H), 7.66 (s, 1H), 7.63 (s, 1H), 7.25 (d, 1H), 7.22 (d, 1H), 7.13 (d, 1H), 7.04 (s, 2H), 6.71 (dd, 1H), 5.95 (m, 2H), 3.73 (s, 3H), 3.51 (q, 2H), 2.89 (t, 2H), 2.36 (s, 3H), 2.04 (s, 3H).

MS (ESI⁺) *m*/*z* 427.3 [M + H]⁺.

### Example 30

### N²-[2-(5-methoxy-1H-indol-3-yl)ethyl]-N⁴-(2-methyl-1H-indol-5-yl)-6-(4-methylpiperazin-1-yl)pyrimidine-2,4-diamine

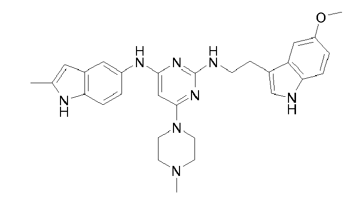

¹H NMR (500 MHz, DMSO-*d*₆, 75°C) *δ* 10.51 (s, 1H), 10.44 (s, 1H), 7.94 (s, 1H), 7.53 (s, 1H), 7.23 (d, 1H), 7.13 (d, 1H), 7.10 (d, 1H), 7.05-7.02 (m, 2H), 6.72 (dd, 1H), 5.97 (s, 1H), 5.79 (t, 1H), 5.26 (s, 1H), 3.75 (s, 3H), 3.55 (q, 2H), 3.39 (t, 4H), 2.94 (t, 2H), 2.35 (s, 3H), 2.32 (t, 4H), 2.20 (s, 3H).

MS (ESI⁺) *m*/*z* 511.5 [M + H]⁺.

### Intermediate 31

### N-(1H-indol-5-yl)-2-chloropyrimidin-4-amine

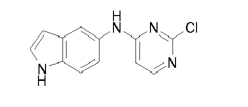

¹H NMR (500 MHz, DMSO-*d*₆) *δ* 11.12 (s, 1H), 9.81 (s, 1H), 8.04 (d, 1H), 7.67 (br. s, 1H), 7.40 (d, 1H), 7.36 (t, 1H), 7.11 (br. s, 1H), 6.61 (br. s, 1H), 6.42 (s, 1H). MS (ESI⁺) *m*/*z* 245.3 [M + H]⁺.

### Intermediate 32

### N-(2-methyl-1H-indol-5-yl)-2-chloropyrimidin-4-amine

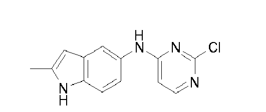

¹H-NMR (500 MHz, DMSO-*d*₆, 75°C) *δ* 10.76 (s, 1H), 9.54 (s, 1H), 8.01 (d, 1H), 7.47 (s, 1H), 7.26 (d, 1H), 7.01 (d, 1H), 6.56 (d, 1H), 6.11 (s, 1H), 2.39 (s, 3H). MS (ESI⁺) m/z 259.1 [M + H]⁺.

### Intermediate 33

### N-(2-chloropyrimidin-4-yl)-1,2-dimethyl-1H-indol-5-amine

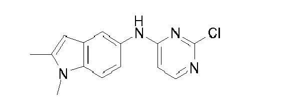

¹H-NMR (500 MHz, DMSO-*d*₆, 75°C) *δ* 9.57 (s, 1H), 8.02 (d, 1H), 7.51 (s, 1H), 7.35 (d, 1H), 7.10 (d, 1H), 6.58 (d, 1H), 6.20 (s, 1H), 3.66 (s, 3H), 2.41 (s, 3H).

MS (ESI⁺) *m*/*z* 273.1 [M + H]⁺.

### Intermediate 34

### N-(2-chloropyrimidin-4-yl)-2,3-dimethyl-1H-indo-1-5-amine

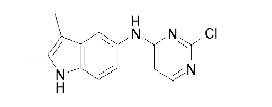

¹H-NMR (500 MHz, DMSO-*d*₆, 75°C) *δ* 10.52 (s, 1H), 9.55 (s, 1H), 8.01 (d, 1H), 7.42 (s, 1H), 7.23 (d, 1H), 7.02 (d, 1H), 6.56 (d, 1H), 2.32 (s, 3H), 2.14 (s, 3H). MS (ESI⁺) m/z 273.2 [M + H]⁺.

### Intermediate 35

### [5-(2-chloropyrimidin-4-ylamino)-1H-indol-2-yl]methanol

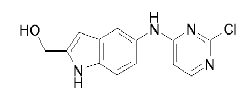

¹H-NMR (500 MHz, DMSO-*d*₆, 75°C) *δ* 10.84 (s, 1H), 9.56 (s, 1H), 8.02 (d, 1H), 7.55 (s, 1H), 7.34 (d, 1H), 7.07 (dd, 1H), 6.58 (d, 1H), 6.27 (s, 1H), 5.01 (m, 1H), 4.62 (d, 2H).

### Intermediate 36

### methyl 5-(2-chloropyrimidin-4-ylamino)-1H-indole-2-carboxylate

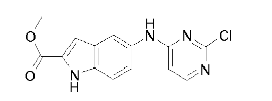

¹H-NMR (500 MHz, DMSO-*d*₆, 75°C) *δ* 11.76 (s, 1H), 9.70 (s, 1H), 8.07 (d, 1H), 7.80 (s, 1H), 7.47 (d, 1H), 7.32 (dd, 1H), 7.14 (s, 1H), 6.65 (d, 1H), 3.89 (s, 3H). MS (ESI⁺) *m*/*z* 303.1 [M + H]⁺.

### Intermediate 37

### N-(2-chloropyrimidin-4-yl)-1H-benzo[d]imidazol-5-amine

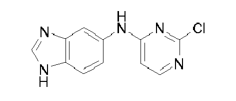

¹H NMR (500 MHz, DMSO-*d*₆) *δ* 12.38 (br s, 1H), 10.00 (br s, 1H), 8.19 (s, 1H), 8.10 (d, 1H), 7.94 (br s, 1H), 7.58 (d, 1H), 7.22 (d, 1H), 6.71 (d, 1H).

MS (ESI⁺) *m*/*z* 246.1 [M + H]⁺.

### Intermediate 38

### N-(2-chloropyrimidin-4-yl)-2-methyl-1H-benzo[d]imidazol-5-amine

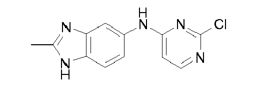

¹H-NMR (500 MHz, DMSO-*d*₆, 75°C) *δ* 12.01 (s, 1H), 9.72 (m, 1H), 8.07 (s, 1H), 7.72 (m, 1H), 7.46-7.36 (m, 1H), 7.18-7.11 (m, 1H), 6.66 (m, 1H), 2.48 (s, 3H). MS (ESI⁺) *m*/*z* 260.1 [M + H]⁺.

### Intermediate 39

### N-(2-chloropyrimidin-4-yl)-1H-indol-6-amine

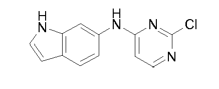

¹H-NMR (500 MHz, DMSO-*d*₆) *δ* 11.13 (s, 1H), 9.95 (s, 1H), 8.08 (d, 1H), 7.77 (br s, 1H), 7.50 (d, 1H), 7.31 (s, 1H), 7.03 (d, 1H), 6.71 (d, 1H), 6.39 (d, 1H).

MS (ESI⁺) *m*/*z* 245.1 [M + H]⁺.

### Intermediate 40

### N-(2-chloropyrimidin-4-yl)-1H-indol-4-amine

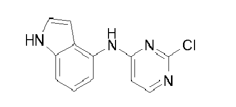

¹H-NMR (500 MHz, DMSO-*d*₆, 75°C) *δ* 11.06 (br s, 1H), 9.64 (s, 1H), 8.08 (d, 1H), 7.31 (t, 1H), 7.29-7.25 (m, 2H), 7.10 (t, 1H), 6.67 (d, 1H), 6.48 (s, 1H).

MS (ESI⁺) *m*/*z* 245.1 [M + H]⁺.

### Intermediate 41

### N-[2-(1H-indol-3-yl)ethyl]-2-chloropyrimidin-4-amine

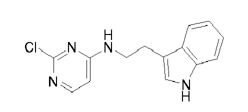

¹H-NMR (500 MHz, DMSO-*d*₆, 75°C) *δ* 10.66 (s, 1H), 7.90 (d, 1H), 7.79 (br s, 1H), 7.59 (d, 1H), 7.35 (d, 1H), 7.15 (s, 1H), 7.07 (t, 1H), 6.99 (t, 1H), 6.44 (d, 1H), 3.56 (m, 2H), 2.96 (t, 2H).

MS (ESI⁺) *m*/*z* 273.2 [M + H]⁺.

### Intermediate 42

### 2-chloro-N-[2-(5-methoxy-1H-indol-3-yl)ethyl]pyrimidin-4-amine

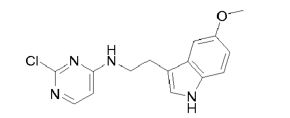

¹H-NMR (500 MHz, DMSO-*d*₆,75°C) *δ* 10.50 (s, 1H), 7.90 (d, 1H), 7.79 (br s, 1H), 7.24 (d, 1H), 7.11 (s, 1H), 7.04 (s, 1H), 6.73 (dd, 1H), 6.45 (d, 1H)3.78 (s, 3H), 3.56 (br s, 2H), 2.93 (t, 2H).

MS (ESI⁺) *m*/*z* 303.1 [M + H]⁺.

### Intermediate 43

### N-(2-methyl-1H-indol-5-yl)-2-chloro-6-methylpyrimidin-4-amine

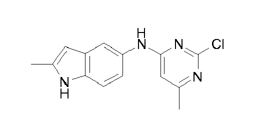

¹H NMR (500 MHz, CD₃OD) *δ* 7.43 (br. s, 1H), 7.26 (d, 1H), 6.98 (br. s, 1H), 6.34 (s, 1H), 6.02 (s, 1H), 2.41 (s, 3H), 2.22 (s, 3H).

MS (ESI⁺) *m*/*z* 273.2 [M + H]⁺.

### Intermediate 44

### 2-chloro-6-(2-methyl-1H-indol-5-ylamino)pyrimidine-4-carboxamide

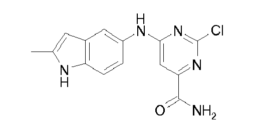

¹H-NMR (500 MHz, DMSO-*d*₆,105°C) *δ* 10.68 (s, 1H), 9.76 (s, 1H), 7.50 (m, 3H), 7.29 (d, 1H), 7.15 (s, 1H), 7.05 (d, 1H), 6.13 (s, 1H), 2.36 (s, 3H).

MS (ESI⁺) *m*/*z* 302.1 [M + H]⁺.

### Intermediate 45

### N-(2-chloro-5-methylpyrimidin-4-yl)-2-methyl-1H-indol-5-amine

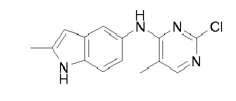

¹H-NMR (500 MHz, DMSO-*d*₆, 75°C) *δ* 10.71 (s, 1H), 8.56 (s, 1H), 7.92 (s, 1H), 7.52 (d, 1H), 7.24 (d, 1H), 7.12 (dd, 1H), 6.11 (s, 1H), 2.39 (s, 3H), 2.15 (s, 3H). MS (ESI⁺) *m*/*z* 273.1 [M + H]⁺.

### Intermediate 46

### 4-chloro-N-[2-(5-methoxy-1H-indol-3-yl)ethyl-6-(4-methylpiperazin-1-yl)pyrimidin-2-amine

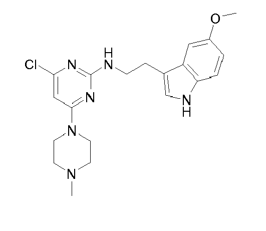

¹H-NMR (500 MHz, DMSO-*d*₆) *δ* 10.62 (s, 1H), 7.22 (d, 1H), 7.13-6.96 (m, 3H), 6.71 (dd, 1H), 6.06 (br s, 1H), 3.75 (s, 3H), 3.55 (br s, 4H), 3.46 (q, 2H), 2.87 (t, 2H), 2.31 (t, 4H), 2.19 (m, 3H).

MS (ESI⁺) *m*/*z* 401.2 [M + H]⁺.

### Biological Assays

The Fluorometric Microculture Cytotoxicity Assay, FMCA, is a three day non-clonogenic microplate-based cell viability assay used for measurement of the cytotoxic and/or cytostatic effect of compounds *in vitro* (Lindhagen, E., et al. Nat Protoc, 2008. 3(8): p. 1364-9). FMCA (Larsson, R. and P. Nygren, Anticancer Res, 1989. 9(4): p. 1111-9) represents a valuable method to measure cytotoxicity in a number of cell types, both cell lines and primary cells from patients (Larsson, R., et al., Int J Cancer, 1992. 50(2): p. 177-85; Fridborg, H., et al., Eur J Cancer, 1999. 35(3): p. 424-32; Dhar, S., et al., Br J Cancer, 1996. 74(6): p. 888-96).

FMCA is based on the principle that fluorescein diacetate (FDA) is converted to the fluorescent probe fluorescein by esterases in the plasma membranes of living cells. For experiments, 96 or 384-well microplates are prepared with compounds and stored at -70°C until use. Cells are then seeded into the drug-prepared plates and placed in an incubator for 72 h. On the last day of incubation, the plates are washed and a buffer containing FDA is added and incubated with the cells for 45 minutes. Finally the fluorescence per well is measured in a fluorometer and a Survival Index % (SI) for each compound-treated well is calculated with the equation: Compound-treated cells minus blank divided by control cells minus blank. A high SI-value indicates a large percentage of living cells and vice versa.

For experiments with compounds of the invention, 96-well plates were prepared as follows:
Compounds were dissolved in DMSO to 10 mM and stored at -20 °C. 96-well plates were prepared with 297 µl of sterile PBS added to each well. The test compounds were thawed, protected from light, mixed, and 3 µl stock solution was added to the 96-well plate to give the concentration 100 µM. Then, an assay plate was prepared by transferring 20 µl of compound solution to a V-bottomed 96-well plate. Compounds at 100 µM were diluted with PBS to 10 µM, and an assay plate containing 20 µl was prepared. The plates were stored at ― 70 °C until use.

On the day of cell seeding, 180 µl of cell suspension was added to each well in the two assay plates. The final concentration of compounds tested was thus 10 µM and 1 µM.

In subsequent experiments, compounds were tested, along with a number of approved cancer drugs (erlotinib, gefitinib, imatinib, dasatinib, pazopanib, sorafenib, and sunitinib) as described above. Initially, the acute lymphoblastic leukemia cell line CCRF-CEM was used throughout. In the assay plates, medium was added to six empty wells (blank wells) and wells were filled with PBS and cell suspension and served as control wells. SI-values were then calculated for each compound-treated well as described above. All experiments were conducted twice and a new batch of plates was prepared for each experiment. The data obtained showed the activity of the example compounds compared to the comparative compounds.

The compounds of the invention are also tested on cancer cell lines HCT116, MCF7, CCRF-CEM, CEM/VM1, H69, H69AR, RPMI8226, 8226/DOX40, U937, U937-VCR, A2780, A2780/Adr, A2780/Cis, and ACHN.

For dose-response experiments, 384-well plates were prepared as follows: Compounds of the invention as well comparative kinase inhibitors sorafenib sunitinib, dasatinib, and pazopanib (reference compounds) were diluted with PBS to a concentration ten-times higher than the desired starting concentration. Then, a Biomek 2000 liquid handling system was employed to serially dilute the compounds in a deep-well 384-well plate. From this plate, assay plates containing 5 µl compound per well were prepared with the Biomek 2000. Certain compounds precipitated when diluted with PBS, and these compounds were therefore prepared in a 96-well plate manually as described above using culture medium RPMI 1640 instead of PBS.

The compounds were also tested at five concentrations, with five times serial dilution on the following cell types: CCRF-CEM, hTERT-RPE1 (normal retinal epithelial cells), hRPTEpiC (normal renal cells), and peripheral blood mononuclear cells (PBMC). Each experiment was performed three times, except for PBMC and hRPTEpiC, which were performed twice. SI-values were calculated, graphs were plotted using GraphPadPrism 5.0 (GraphPad Software Inc. La Jolla, CA) and IC₅₀-values for each cell type and compound were determined from the curves.

The example compounds of the invention were active in the CCRF-CEM cell measurements, showing IC₅₀ values less than 10 µM. Preferred compounds of the invention had IC₅₀ values less than 1 µM. More preferred compounds of the invention had IC₅₀ values less than 10 nM. Sorafenib, sunitinib, dasatinib, and pazopanib had IC₅₀ values of 8.3 µM, 14.1 µM, 9.7 µM, and 25.9 µM respectively, in this assay. Most of the compounds of the invention showed lower IC₅₀ values than the reference compounds and the primary results also showed that the compounds of the invention exhibited an enhanced selectivity towards CCRF-CEM cells, compared to the tested peripheral blood mononuclear cells (PBMC).

Example compounds were further tested in a tubulin polymerization assay kit from Cytoskeleton Inc (Denver, CO, USA). Polymerization is followed by fluorescence enhancement due to the incorporation of a fluorescent reporter into microtubules as polymerization occurs. Vincristine and paclitaxel at 3 µM were used as positive controls for tubulin polymerization inhibition and stabilisation, respectively. All compounds were dissolved in DMSO, which was used as solvent control. For experiments, example compounds and control compounds were incubated with bovine tubulin protein in a cell-free environment and the fluorescence was then measured in a fluorometer (Fluostar Optima, BMG Labtech, Offenburg, Germany) at 360/450 nm every minute for a total of 60 mins. Selected example compounds were tested, showing inhibitory effects on tubulin polymerization.

Example compounds were further tested for induction of apoptosis in a Live cell imaging setup. The NucView™ 488 Caspase-3 Assay Kit for Live cells (Biotium, Inc. Hayward, CA, USA) was used. HCT116 cells were plated the day before the experiment in black glass-bottom PerkinElmer plates and compounds with chosen concentrations were then added. Finally, the DEVD-NucView488 Caspase-3 substrate was added and the plate was placed in an IncuCyteFLR for live-cell imaging. When the substrate is cleaved by activated caspase-3, a dye is released which becomes fluorescent upon binding to DNA (Cen H et al. DEVD-NucView488: a novel class of enzyme substrates for real-time detection of caspase-3 activity in live cells. FASEB J. 2008 Jul;22(7):2243-52.). Staurosporin at 1 µM was used as a positive control for apoptosis. Selected example compounds were tested and all of them induced apoptosis at the chosen concentrations at varying time points.

## Claims

1. A compound of formula I or a pharmaceutically acceptable ester, amide,
solvate or salt thereof, 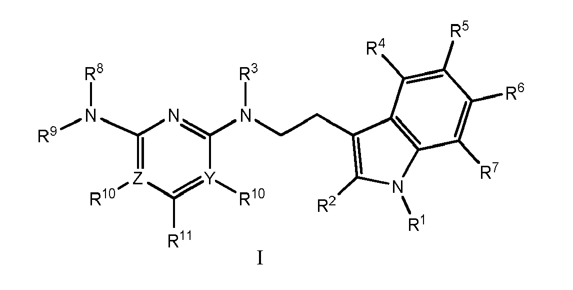 wherein
Z represents carbon or nitrogen;
Y represents carbon or nitrogen, wherein one of Z and Y represents nitrogen; R¹, R³, and R⁸ are independently selected from hydrogen and (C₁-C₄)alkyl; R² is selected from hydrogen, (C₁-C₄)alkyl, (C₁-C₄)alkyl-NH(C₁-C₄)alkyl, (C₁-C₄)alkyl-N[(C₁-C₄)alkyl]₂, and (CO)OH;
R⁴, R⁵, R⁶, and R⁷ are independently selected from hydrogen, halogen, hydroxy, amino, nitro, cyano, (C₁-C₄)alkyl, (C₁-C₄)alkyl-OH, (C₁-C₄)alkyl-NH₂, (C₁-C₄)alkyl-NH(C₁-C₄)alkyl, (C₁-C₄)alkyl-N[(C₁-C₄)alkyl]₂, (C₁-C₄)alkyl-NH(CO)(C₁-C₄)alkyl, (CO)OH, (CO)NH₂, (CO)NH(C₁-C₄)alkyl, (CO)N[(C₁-C₄)alkyl]₂, O(C₁-C₄)alkyl, O(C₁-C₄)alkyl(C₂-C₅)heterocyclyl, O(C₁-C₄)alkyl(C₂-C₅)heterocyclyl(C₁-C₄)alkyl, O(C₁-C₄)alkyl(CO)OH, O(C₁-C₄)alkyl(CO)NH(C₁-C₄)alkyl, O(C₁-C₄)alkyl(CO)N[(C₁-C₄)alkyl]₂, OCF₃, NH(C₁-C₄)alkyl, N[(C₁-C₄)alkyl]₂, NH(CO)(C₁-C₄)alkyl, NHSO₂(C₁-C₄)alkyl, N[(C₁-C₄)alkyl]SO₂(C₁-C₄)alkyl, SH, S(C₁-C₄)alkyl, SO₂NH₂, SO₂NH(C₁-C₄)alkyl, and SO₂N[(C₁-C₄)alkyl]₂;
R¹⁰ is selected from hydrogen, amino, and (C₁-C₄)alkyl when Z or Y is carbon;
R¹¹ is selected from hydrogen, amino, (C₁-C₄)alkyl, (C₁-C₄)alkyl(C₂-C₅)heterocyclyl, (C₁-C₄)alkyl(C₂-C₅)heterocyclyl(C₁-C₄)alkyl, (CO)OH, (CO)NH₂, (CO)NH(C₁-C₄)alkyl, (CO)N[(C₁-C₄)alkyl]₂, (CO)(C₁-C₄)alkyl, (C₂-C₅)heterocyclyl, (C₂-C₅)heterocyclyl(C₁-C₄)alkyl, NH(C₁-C₄)alkyl, N[(C₁-C₄)alkyl]₂, NH(CO)(C₁-C₄)alkyl, NHSO₂(C₁-C₄)alkyl, N[(C₁-C₄)alkyl]SO₂(C₁-C₄)alkyl, SO₂NH₂, SO₂NH(C₁-C₄)alkyl, and SO₂N[(C₁-C₄)alkyl]₂;
R⁹ represents 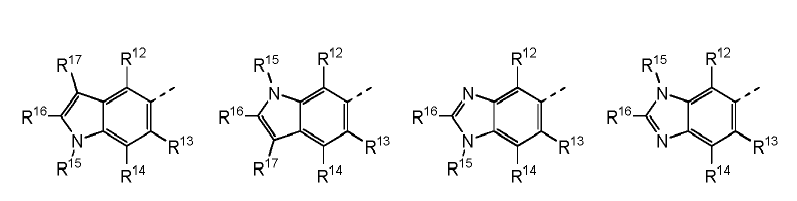 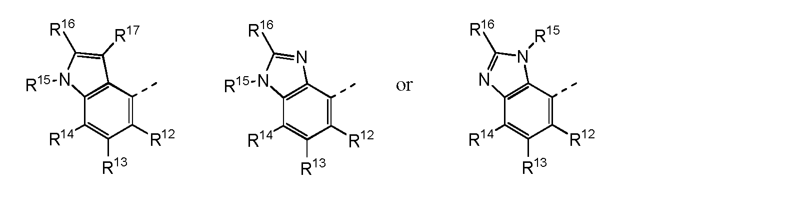 _{R}¹², R¹³, and R¹⁴ are independently selected from hydrogen, halogen, hydroxy, (C₁-C₄)alkyl, O(C₁-C₄)alkyl, NH(C₁-C₄)alkyl, and N[(C₁-C₄)alkyl]₂; R¹⁵ is selected from hydrogen and (C₁-C₄)alkyl; and
R¹⁶ and R¹⁷ are independently selected from hydrogen, halogen, (C₁-C₄)alkyl, (C₁-C₄)alkyl(C₂-C₅)heterocyclyl, (C₁-C₄)alkyl(C₂-C₅)heterocyclyl(C₁-C₄)alkyl, (C₁-C₄)alkyl(CO)OH, (C₁-C₄)alkyl(CO)NH₂, (C₁-C₄)alkyl(CO)NH(C₁-C₄)alkyl, (C₁-C₄)alkyl(CO)N[(C₁-C₄)alkyl]₂, (C₁-C₄)alkyl-OH, (C₁-C₄)alkyl-O(C₁-C₄)alkyl, (C₁-C₄)alkyl-NH(C₁-C₄)alkyl, (C₁-C₄)alkyl-N[(C₁-C₄)alkyl]₂, (C₁-C₄)alkyl-NH(CO)(C₁-C₄)alkyl, (CO)OH, (CO)NH₂, (CO)NH(C₁-C₄)alkyl, (CO)N[(C₁-C₄)alkyl]₂, (CO)(C₁-C₄)alkyl, (CO)(C₂-C₅)heterocyclyl, and (CO)(C₂-C₅)heterocyclyl)(C₁-C₄)alkyl.

2. A compound according to claim 1, wherein R¹, R², R³, and R⁸ represent hydrogen.

3. A compound according to claim 1 or 2, wherein R⁴, R⁵, R⁶, and R⁷ are independently selected from hydrogen, halogen, hydroxy, (C₁-C₄)alkyl, O(C₁-C₄)alkyl, O(C₁-C₄)alkyl(C₂-C₅)heterocyclyl, and OCF₃.

4. A compound according to any one of claims 1 to 3, wherein R¹¹ is selected from hydrogen, (C₁-C₄)alkyl, (CO)NH₂, and (C₂-C₅)heterocyclyl(C₁-C₄)alkyl.

5. A compound according to any one of claims 1 to 4, wherein R⁹ is selected
from

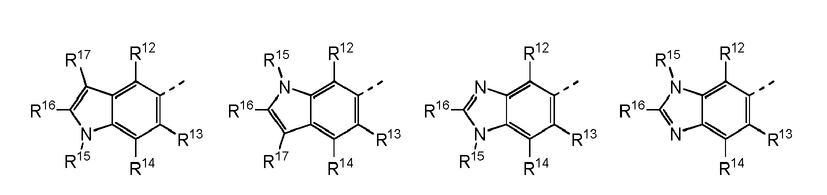

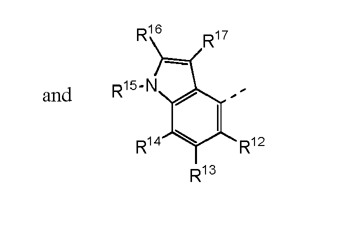

6. A compound according to any one of claims 1 to 5, wherein R¹², R¹³ and R¹⁴ represent hydrogen.

7. A compound according to any one of claims 1 to 6, wherein R¹⁵ is selected from hydrogen, and methyl.

8. A compound according to any one of claims 1 to 7, wherein R¹⁶ and R¹⁷ are independently selected from hydrogen, (C₁-C₄)alkyl, (C₁-C₄)alkyl-OH, and (CO)OH.

9. A compound according to claim 1, wherein
Z represents carbon and Y represents nitrogen;
R¹ R², R³, R⁸, R¹², R¹³, and R¹⁴ represent hydrogen;
R⁴, R⁵, R⁶, and R⁷ are independently selected from hydrogen, halogen, hydroxy, (C₁-C₄)alkyl, O(C₁-C₄)alkyl, O(C₁-C₄)alkyl(C₂-C₅)heterocyclyl, and OCF₃;
R¹⁰ is selected from hydrogen and (C₁-C₄)alkyl when Z or Y is carbon;
R¹¹ is selected from hydrogen, (C₁-C₄)alkyl, (CO)NH₂, and (C₂-C₅)heterocyclyl(C₁-C₄)alkyl;
R⁹ is selected from 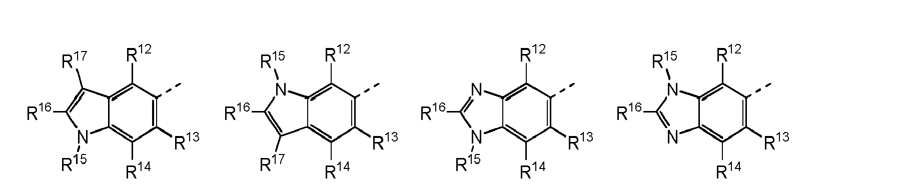 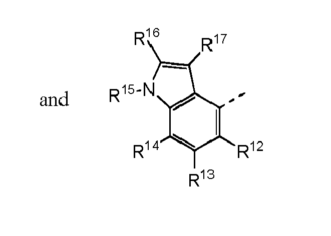 R¹⁵ is selected from hydrogen and methyl;
and R¹⁶ and R¹⁷ are independently selected from hydrogen, (C₁-C₄)alkyl, (C₁-C₄)alkyl-OH, and (CO)OH.

10. A compound according to claim 9, wherein R¹⁶ represents methyl.

11. A compound according to claim 9 or 10, wherein R⁵ is selected from methoxy, methyl, and fluoro.

12. A compound according to claim 1, said compound being selected from:
*N²*-[2-(1*H*-indol-3-yl)ethyl]-*N⁴*-(1*H*-indol-5-yl)pyrimidine-2,4-diamine;
*N*⁴-(1*H*-indol-5-yl)-*N*²-[2-(5-methoxy-1*H*-indol-3-yl)ethyl]pyrimidine-2,4-diamine;
*N²-*[2-(1*H-*indol-3- yl)ethyl]-*N⁴*-(2-methyl-1*H*-indol-5-yl)pyrimidine-2,4-diamine;
*N²*-[2-(5-methoxy-1*H*-indol-3-yl)ethyl]-*N⁴*-(2-methyl-1*H*-indol-5-yl)pyrimidine-2,4-diamine;
*N²*-[2-(5-ethoxy-1*H-*indol-3-yl)ethyl]-*N⁴*-(2-methyl-1*H-*indol-5-yl)pyrimidine-2,4-diamine;
*N*⁴-(2-methyl-1*H*-indol-5-yl)-*N*²- {2-[5-(2-morpholinoethoxy)-1*H*-indol-3-yl]ethyl}pyrimidine-2,4-diamine;
*N*⁴-(2-methyl-1*H-*indol-5-yl)-*N*²-{2-[5-(trifluoromethoxy)-1*H*-indol-3-yl]ethyl}pyrimidme-2,4-diamme;
3- {2-[4-(2-methyl-1*H*-indol-5-ylamino)pyrimidin-2-ylamino]ethyl}-1*H*-indol-5-ol;
*N*²-[2-(5-methyl-1*H*-indol-3-yl)ethyl]-*N*⁴-(2-methyl-1*H*-indol-5-yl)pyrimidine-2,4-diamine;
*N*²-[2-(5-fluoro-1*H-*indol-3-yl)ethyl]-*N*⁴-(2-methyl-1*H-*indol-5-yl)pyrimidine-2,4-diamine;
*N*²-[2-(6-methoxy-1*H*-indol-3-yl)ethyl]-*N*⁴-(2-methyl-1*H*-indol-5-yl)pyrimidine-2,4-diamine;
*N*²-[2-(7-methoxy-1*H*-indol-3-yl)ethyl]-*N*⁴-(2-methyl-1*H*-indol-5-yl)pyrimidine-2,4-diamine;
*N*⁴-(1,2-dimethyl-1*H-*indol-5-yl)-*N*²-[2-(5-methoxy-1*H-*indol-3-yl)ethyl]pyrimidine-2,4-diamine;
*N*⁴-(2,3-dimethyl-1*H*-indol-5-yl)-*N*²-[2-(5-methoxy-1*H*-indol-3-yl)ethyl]pyrimidine-2,4-diamine;
(5- {2-[2-(5-methoxy-1*H*-indol-3-yl)ethylamino]pyrimidin-4-ylamino}-1*H-*indol-2-yl)methanol;
methyl 5- {2-[2-(5-methoxy-1*H*-indo-1-3-yl)ethylamino]pyrimidm-4-ylamino}-1*H*-indole-2-carboxylate;
2-hydroxyethyl 5- {2-[2-(5-methoxy-1*H*-indol-3-yl)ethylamino]pyrimidin-4-ylamino}-1*H*-indole-2-carboxylate;
*N*⁴*-*(1*H-*benzo[*d*]imidazol-5-yl)-*N*²-[2-(5-methoxy-1*H*-indol-3-yl)ethyl]pyrimidine-2,4-diamine;
*N*²-[2-(5-methoxy-1*H*-indol-3-yl)ethyl]-*N*⁴-(2-methyl-1*H*-benzo[*d*]imidazol-5-yl)pyrimidine-2,4-diamine;
*N*⁴*-*(1*H-*indol-6-yl)-*N*²-[2-(5-methoxy-1*H*-indol-3-yl)ethyl]pyrimidine-2,4-diamine;
*N*⁴*-*(1*H-*indol-4-yl)-*N*²-[2-(5-methoxy-1*H*-indol-3-yl)ethyl]pyrimidine-2,4-diamine;
*N*⁴*-*[2-(1*H-*indol-3-yl)ethyl]-*N*²-(2-methyl-1*H*-indol-5-yl)pyrimidine-2,4-diamine;
*N*⁴-[2-(5-methoxy-1*H*-indol-3-yl)ethyl]-*N*²-(2-methyl-1*H*-indol-5-yl)pyrimidine-2,4-diamine;
*N*⁴*-*[2-(1*H-*indol-3-yl)ethyl]-*N*²-(1*H-*indol-6-yl)pyrimidine-2,4-diamine;
*N*⁴*-*[2-(1*H* indol-3-yl)ethyl]-*N*²-(1*H*-indol-4-yl)pyrimidine-2,4-diamine;
*N²*-[2-(1*H*-indol-3-yl)ethyl]-6-methyl-*N⁴*-(2-methyl-1*H*-indol-5-yl)pyrimidine-2,4-diamine;
*N*²-[2-(5-methoxy-1*H*-indol-3-yl)ethyl]-6-methyl-*N*⁴-(2-methyl-1*H*-indol-5-yl)pyrimidine-2,4-diamine;
2-[2-(5-methoxy-1*H-*indol-3-yl)ethylamino]-6-(2-methyl-1*H-*indol-5-ylamino)pyrimidine-4-carboxamide;
*N*²-[2-(5-methoxy-1*H-*indol-3-yl)ethyl]-5-methyl-*N*⁴-(2-methyl-1*H-*indol-5-yl)pyrimidine-2,4-diamine; and
*N*²-[2-(5-methoxy-1*H*-indol-3-yl)ethyl]-*N*⁴-(2-methyl-1*H*-indol-5-yl)-6-(4-methylpiperazin-1-yl)pyrimidine-2,4-diamine.

13. A compound according to any one of claims 1 to 12, for use in therapy.

14. A compound according to any one of claims 1 to 12, for use in treatment of cancer.

15. A pharmaceutical composition comprising a compound according to any one of claims 1 to 12 together with pharmaceutically acceptable diluents and carriers.
